# EUROPEAN PATENT SPECIFICATION

(11) **EP 4 034 037 B1**
(45) Date of publication and mention of the grant of the patent: **03.07.2024**
(21) Application number: 20796477.6
(22) Date of filing: 23.09.2020
(51) Int. Cl.: A61F 2/07, A61F 2/24, A61F 2/856

(54) **ARTIFICIAL CARDIAC VALVE**
KÜNSTLICHE HERZKLAPPE
VALVULE CARDIAQUE ARTIFICIELLE

(30) Priority: 26.09.2019 EP 19199843
(43) Date of publication of application: 03.08.2022
(73) Proprietor: Biotronik AG, 8180 Bülach (CH)
(72) Inventor: NOLLERT, Georg, 82064 Strasslach (DE); KISSLING, Tobias, 3005 Bern (CH)
(74) Representative: Biotronik Corporate Services SE
(86) International application number: PCT/EP2020/076483
(87) International publication number: WO 2021/058512

(56) References cited:
- EP-A1- 2 929 860
- US-A1- 2013 122 248
- US-A1- 2018 021 130

## Description

The present invention relates to an artificial cardiac valve according to claim 1. Such an artificial cardiac valve can be used in assisting or replacing tricuspid valve functionality or mitral valve functionality.

WO 2017/147082 A1 discloses a bifurcated tubular graft for treating tricuspid regurgitation. Thereby, one leg of the bifurcated graft extends across the tricuspid valve. This leg comprises in its interior a bioprosthetic valve that replaces the functionality of the tricuspid valve. However, the implantation of such bifurcated graft is rather difficult and its method of manufacture is rather complex.

CN 206995366 U describes a stent for treating tricuspid regurgitation. This stent has an overall oblong shape and is placed with one end in the inferior vena cava and with its other end in the superior vena cava. It comprises a central opening to be placed in the right atrium and allowing an unhindered blood flow into an interior of the stent and out of the interior of the stent. The stent furthermore comprises two biological cardiac valves placed in the interior of the stent. These two biological valves allow a blood flow from the inferior vena cava and from the superior vena cava to the right atrium, but not in the opposite direction. To achieve correct positioning of the stent within the heart of the patient, it comprises a plurality of radiopaque markers that mark the position of the central opening of the stent. The configuration of the above-described device appears to be rather complex.

CN 207253400 U also describes a stent assembly for the treatment of tricuspid regurgitation. This stent also has an overall oblong shape. It comprises two grid-like cylindrical stents that are to be implanted into the inferior vena cava and into the superior vena cava. Between these two grid-like cylindrical stents, a connecting wire is provided that prevents a displacement of the cylindrical stents. The connecting wire does not limit a blood flow in the area in which it is positioned. In order to prevent an undesired blood flow from the right atrium into the inferior vena cava and the superior vena cava, both grid-like cylindrical stents comprise in each case a biological valve sewn to the respective stent. The configuration of the above-described device appears to be rather complex as well.

US 2018/0021130 A1 describes a tricuspid insufficiency treatment device including a vena cava member and that is implantable in a vena cava of a patient. Therein, the vena cava member is formed with a fenestration and further includes a blocking member arranged to block and unblock the fenestration.

It is an object of the present invention to provide an artificial cardiac valve that can assist or replace tricuspid valve functionality or mitral valve functionality and that can be more easily manufactured and/or more easily implanted than artificial cardiac valves known from prior art.

This object is achieved with an artificial cardiac valve having the claim elements according to claim 1.

In general, the present invention relates to an artificial cardiac valve comprising a support structure and a covering, the covering at least partially enwrapping an outer side of the support structure, and wherein the covering comprises a longitudinal opening, and wherein different sections of the covering are - due to the longitudinal opening - relatively movable to each other and either overlap or tightly adjoin to one another along the longitudinal opening, so that the covering can be present in two different configurations, an open and a closed configuration acting as a check valve.

Thus, such a cardiac valve comprises a support structure such as a hollow-cylindrical or tubular stent and a covering at least partially enwrapping an outer side of the support structure (e.g. stent). In one embodiment, the covering is enwrapping an outer side of the support structure (e.g. stent). The support structure, preferably being a hollow-cylindrical or tubular stent, has a first end area and a second end area, whereby the second end area is spaced apart from the first end area.

With the context of the present invention and throughout the description portion, the expression "support structure" may be interchangeably used with a "stent", a "stent frame", or, e.g., a "stent structure", or vice versa. Preferably, the expressions "support structure" and "stent", "stent frame", "stent structure" relate in shape and nature to a hollow-cylindrical or tubular stent, stent frame or stent structure with the context of the invention. In one embodiment of the invention, in view of the aforementioned "at least partially enwrapping of an outer side of the support structure", the first end area and/or the second end area may be free of the covering.

In accordance with the invention, the support structure further has an intermediate area extending between the first end area and the second end area along the longitudinal axis (L) of the stent, whereby, the second end area is spaced apart from the first end area.

In one embodiment of the invention, the covering is connected to the support structure by at least one connection. Said connection can be a fixed connection (e.g. a tight connection) or a loosely connection, or a combination of both as the skilled person readily understands in dependence of whatever specifications of the artificial cardiac valve shall be met.

Thus, in an alternative embodiment of the invention, the covering is loosely attached to the support structure by at least one loosely connection. With this context, "loosely attached" or the like means that the covering is not tightly connected over an entire length of the support structure.

The connection between the covering and the support structure can be realized, e.g., by sewing, gluing, suitable material embedding techniques, electrospinning or any other appropriate fixation methods known by the skilled artisan. A a suitable material embedding technique means that at least a part of the support structure is embedded in a part of the covering or in an extension thereof, so as to connect the covering to the support structure. Particularly, the covering is connected to the support structure in a sutureless (seamless) fashion. A part of the three-dimensional shape of the covering thereby deviates from the three-dimensional shape of the support element. The support element is not embedded in the part of the covering that deviates from the support structure. As a result, this part of the covering is free to move independently from the support structure and is therefore able to perform a valve function as disclosed herein. This is important, for example, for a heart valve implant in which at least a part of the covering with a longitudinal opening as functional element, i.e. the heart valve element, has to be able to flexibly open and close in response to antegrade and retrograde blood flow. Preferably, at least 10% of the three-dimensional shape of the covering deviates from the support structure, more preferably at least 20%, even more preferably at least 30%, yet more preferably at least 40% and most preferably at least 50%. The larger the part of the covering that deviates from the support structure, the more of the covering surface area can be used by the implant to perform a valve function that necessitates movement of the longitudinal opening that is independent of that of the support structure.

Preferably, the covering comprises at least a part that is essentially made of bacterial cellulose. This part of the covering preferably has a part of the support structure embedded within so as to connect the covering to the support structure.

Following the above, in one embodiment of the invention the artificial cardiac valve comprises:
- a support structure as disclosed herein, and
- a covering as disclosed herein,
wherein the three-dimensional shape of the covering at least in part deviates from the three-dimensional shape of the support structure, and wherein a part of the support structure is embedded in a part of the covering that comprises bacterial cellulose so as to connect the covering to the support structure.

A structure that is essentially made of bacterial cellulose in the context of this disclosure refers to a structure that is made by deposition of bacterial cellulose on a given surface by a bacterium that produces bacterial cellulose. Such bacterial cellulose is usually of high purity and therefore the thus formed object essentially consists of bacterial cellulose. Bacterial cellulose that contains impurities or for example cellular debris from bacteria or entire bacteria that were incorporated during the deposition of the bacterial cellulose by the bacteria would still be counted in the context of this disclosure as bacterial cellulose that is essentially made of, or essentially consists of, bacterial cellulose.

The covering of the invention fluidly separates an interior of the support structure from an outer space or an outer side of the support structure. I.e., fluid such as blood being present in the support structure cannot flow from the interior towards the outside of the support structure (antegrade flow), as long as the covering does not provide an appropriate opening for such flow such as a longitudinal opening. Likewise, the flow of a fluid is not possible in the inverse direction from an outside into the interior of the support structure without an appropriate opening such as a longitudinal opening in the covering (retrograde flow).

In order to allow a fluid such as blood to enter the interior of the support structure, the first end area comprises a first opening. Likewise, the second end area comprises a second opening. Typically, the first opening and the second opening are provided in an axial terminus of the first end area and the second end area so that blood can enter into the interior of the stent along the longitudinal axis of the stent. However, other designs of the first opening and the second opening are also possible. Generally, the first opening and the second opening are not covered by the covering.

The covering comprises or is essentially made from at least two strip-shaped elements substantially surrounding the support structure and being arranged in such a way that allows for the formation of a longitudinal opening, preferably two longitudinal openings. Thereby, the longitudinal opening(s) extend(s) from a respective longitudinal opening starting point to a respective longitudinal opening ending point. Thereby, the respective longitudinal opening starting point and the respective longitudinal opening ending point are spaced apart from each other along a longitudinal axis (L) of the support structure.

With the context of the present invention, the expression "longitudinal opening" denotes any form of opening that extends in a longitudinal direction. For instance, a longitudinal opening may be realized by the mere arrangement of at least two overlapping strip-shaped elements which form some sort of a gap in between when they are relatively movable to one another. Also, a cut in at least one strip-shaped element forms a longitudinal opening, whereby the cut may be realized by a straight line or in a curved manner. In certain instances with the context of the present invention, the skilled person clearly understands that the expressions "longitudinal opening", "gap", "cut", and "opening" may be used interchangeably.

In one embodiment, the covering comprises or is essentially made from at least two strip-shaped elements having one or more longitudinal openings such as a cut, preferably one or more longitudinal cuts, most preferred two cuts. Thereby, the longitudinal opening(s) such as a cut extend(s) from a respective longitudinal opening starting point to a respective longitudinal opening ending point. Thereby, the respective longitudinal opening starting point and the respective longitudinal opening ending point are spaced apart from each other along the longitudinal axis (L) of the support structure.

In one embodiment of the invention, the covering comprises or is essentially made from at least four strip-shaped elements substantially surrounding the support structure and being arranged in such a way that allows for the formation of a longitudinal opening, preferably two longitudinal openings, more preferably four longitudinal openings. Thereby, the longitudinal opening(s) extend(s) from a respective longitudinal opening starting point to a respective longitudinal opening ending point. Thereby, the respective longitudinal opening starting point and the respective longitudinal opening ending point are spaced apart from each other along a longitudinal axis (L) of the support structure.

In one embodiment, the covering comprises or is essentially made from at least four strip-shaped elements having one or more longitudinal openings such as a cut, preferably one or more longitudinal cuts, most preferred four cuts. Thereby, the longitudinal opening(s) such as a cut extend(s) from a respective longitudinal opening starting point to a respective longitudinal opening ending point. Thereby, the respective longitudinal opening starting point and the respective longitudinal opening ending point are spaced apart from each other along the longitudinal axis (L) of the support structure.

In one embodiment of the invention, the covering comprises or is essentially made from at least six strip-shaped elements substantially surrounding the support structure and being arranged in such a way that allows for the formation of a longitudinal opening, preferably two longitudinal openings, more preferably four longitudinal openings, most preferred six longitudinal openings. Thereby, the longitudinal opening(s) extend(s) from a respective longitudinal opening starting point to a respective longitudinal opening ending point. Thereby, the respective longitudinal opening starting point and the respective longitudinal opening ending point are spaced apart from each other along a longitudinal axis (L) of the support structure.

In one embodiment, the covering comprises or is essentially made from at least six strip-shaped elements having one or more longitudinal openings such as a cut, preferably one or more longitudinal cuts, most preferred six cuts. Thereby, the longitudinal opening(s) such as a cut extend(s) from a respective longitudinal opening starting point to a respective longitudinal opening ending point. Thereby, the respective longitudinal opening starting point and the respective longitudinal opening ending point are spaced apart from each other along the longitudinal axis (L) of the support structure.

With the context of the present invention, the at least two, four, or six strip-shaped elements comprise a region in which the respective longer outer edges of the strip-shaped elements are non-fixedly connected to one another either by an overlap or they adjoin tightly to one another in such a way that substantially no longitudinal opening is generated in between the respective longer outer edges of the strip-shaped element and thus between the support structure and the covering.

In view of the foregoing disclosure, according to the invention the covering may comprise one or more cuts in the strip-shaped element(s) in order to form a longitudinal opening in the covering. Each cut extends from a respective first cut starting point to a respective first cut ending point. Thereby, the first cut starting point and the first cut ending point are spaced apart from each other along the longitudinal axis of the stent.

With the above context, a covering comprising one or more cuts reduces the sewing effort and a strip-shaped element of the invention with more than one cut increases the symmetry of the artificial cardiac valve and increases the yield of the material for the cover. This holds true, for instance, where the cover is made of pericardium, where as a natural product it is not necessarily easy to find a large homogenous area of pericardium that achieves the required specifications over the entire area.

According to the claimed invention, the longitudinal opening such as a cut extends at least partially along the intermediate area of the support structure (i.e., in the area of the covering in which it enwraps the intermediate section of the stent). Furthermore, the longitudinal opening (e.g. a cut) divides the covering into two sections. One of these sections is present on a first side of the longitudinal opening. The other section lies opposite the first section on a second side of the longitudinal opening. These two sections are relatively movable to each other and either overlap along the longitudinal opening or tightly adjoin one another without leaving a substantial gap (e.g. in case of a cut). This results in the covering being able to be present in two configurations, i.e., an open (first configuration) and a closed configuration (second configuration).

In an open configuration (first configuration), a gap is present between the first section of the covering and the second section of the covering. Thereby, the gap allows a blood flow between the interior of the support structure and the outer space or outer side of the support structure across the support structure (e.g. the open cells of a stent) and the gap in the covering. Thus, the gap forms an opening (e.g. a longitudinal opening) in the covering which enables a blood flow across the longitudinal opening of the covering.

In a closed configuration (second configuration), no gap is present between the first section and the second section of the covering. Then, substantially no blood flow between the interior of the support structure and the outer space or the outer side of the support structure across the support structure (e.g. the open cells of a stent) and the gap in the covering is possible.

Typically, the covering is in its open configuration (first configuration) if blood flows from an interior of the support structure to an outer space or an outer side thereof. Likewise, the covering is typically in its closed configuration (second configuration) if blood presses the covering from an outside or from an outer space of the support structure onto the support structure. Thus, the artificial cardiac valve acts like a check valve that enables a blood flow in one direction (namely, from an interior of the support structure to an outer space thereof = antegrade blood flow), but prevents a blood flow in the opposite direction (namely, from an outer space of the support structure to the interior thereof = retrograde blood flow).

The covering can be brought into its first (open) configuration and its second (closed) configuration by a fluid pressure acting onto the covering. While it is possible that one of the first section and the second section can be lifted with respect to the respective other section in case that blood (or another liquid) presses against the covering from the interior of the support structure, no such lifting is possible if blood (or another liquid) presses onto the covering from an outer space of the support structure. Rather, in the latter case, the first section and the second section are tightly pressed onto each other, thus closing the artificial cardiac valve. Alternatively, in case of no overlap of the first and second section both sections tightly adjoin one another in order to substantially close the gap.

With the above context, in case of no overlap of the first and second section it is advantageous that less material for the covering must be spent and this may lead to a smaller profile (including a smaller diameter when crimped) for delivery of the artificial cardiac valve.

If the artificial cardiac valve is intended to be used in assisting or replacing a tricuspid valve, it is implanted to a patient such that the first end area is located in the inferior vena cava and the second end area is located in the superior vena cava of the patient. Then, the artificial cardiac valve extends through the right atrium of the patient's heart and connects the inferior vena cava with the superior vena cava.

If blood flows from the inferior vena cava and/or the superior vena cava into the artificial cardiac valve, it lifts the first section of the covering with respect to the second section and can easily enter the right atrium. If the right ventricle of the patient's heart contracts to pump blood into the pulmonary artery, the tricuspid valve typically prevents a reflux of blood into the right atrium. If the tricuspid valve does not function correctly, blood will enter the right atrium. However, then it presses the first section of the covering onto the second section of the covering or it tightly adjoins the first section of the covering to the second section of the covering so that blood substantially cannot enter the interior of the artificial cardiac valve. Thus, the blood remains in the right atrium and does not flow back (retrograde blood flow) or only in a physiologically acceptable very small amount of blood flow back into the inferior vena cava or the superior vena cava. As a result, congestion of vital organs such as the liver is avoided, which is the root cause of many complications of tricuspid regurgitation. Thus, the artificial cardiac valve - although it is exclusively located in the right atrium and does not extend across the tricuspid valve - takes over the functionality of the tricuspid valve or at least a part of this functionality and counteracts tricuspid regurgitation.

If the artificial cardiac valve is intended to assist or replace mitral valve functionality, it is implanted into the left atrium of the patient's heart such that the first end area is placed into a first pulmonary vein and the second end area is placed into a second pulmonary vein. To give an example, the first end area can be placed into the right upper pulmonary vein, and the second end area can be placed into the left upper pulmonary vein. Likewise, it would be possible to place the first end area into the right lower pulmonary vein and the second end area into the left lower pulmonary vein. It is also possible to place the first end area into the right upper pulmonary vein and the second end area into the right lower pulmonary vein. Further, it would be possible to place the first end area into the left upper pulmonary vein and the second end area into the left lower pulmonary vein. Hereby, the skilled person readily understands that also other suitable configurations exist where to place the first end area and the second end area of one or more of the artificial cardiac valves in accordance with the present invention. For instance, in an alternative embodiment, the present invention also foresees branched stent concepts for the herein disclosed artificial cardiac valve.

If blood flows then through a pulmonary vein into the left atrium, it can simply lift the first covering section with respect to the second covering section and enter the left atrium through the formed gap between the first section and the second section of the covering. After contraction of the left ventricle, blood present in the left ventricle is typically pressed into the aorta. If the mitral valve does not function properly, the blood can re-enter the left atrium. However, it then presses the first section of the covering either against the second section of the covering and thus forming an overlap or the first and second section tightly adjoin one another, and thus closing the gap between both sections. Following this, any backflow of blood into the interior of the artificial cardiac valve through the longitudinal opening in the covering (i.e. retrograde blood flow) is either avoided or at least significantly reduced to physiologically acceptable amounts of blood backflow. Thus, a reflux of blood into the pulmonary veins is prevented. Consequently, the amount of blood that can be pumped into the aorta is significantly increased.

Thereby, it is not necessary nor intended that the artificial cardiac valve crosses the mitral valve. Rather, it is predominantly located within the left atrium and may be also located in the afferent veins.

Since the artificial cardiac valve is to be implanted either predominantly into the right atrium (and then extending into the inferior vena cava and the superior vena cava) or predominantly into the left atrium (and then extending into two pulmonary veins), it can be much more easily implanted than artificial valves known from prior art having a branched shape, e.g., bifurcated shape and extending across the tricuspid valve or the mitral valve, respectively. In one embodiment of the invention, typically no specific orientation of the presently claimed artificial cardiac valve needs to be observed when implementing the same into the patient's heart. Thus, no specific markers need to be present on the artificial cardiac valve in an embodiment.

In an alternative embodiment, however, a specific orientation of the artificial cardiac valve needs to be observed when implanting the same into the patient's heart Thus, in such instance, suitable and specific markers need to be present on the artificial cardiac valve and/or on the delivery catheter.

The presently claimed artificial cardiac valve can also be more easily manufactured than cardiac valves known from prior art. This is due to the fact that it comprises only one valve entity formed by the covering. With this context, "one valve entity" means that the covering together with the support structure forms one valve entity, but its specific arrangement of strip-shaped elements, strips, sections, longitudinal openings, and/or cuts may differ / vary, and thus may result in different / varying configurations of gaps, cuts, longitudinal openings and overlapping and/or tightly adjoining sections of the covering.

With the context of the present invention, in certain instances, the expressions "strip-shaped element(s)", "strip(s)", and "section(s)" may be used interchangeably for the configuration of a covering and a valve entity of the invention.

In contrast, artificial cardiac valves known from prior art often require two valve entities (e.g., one to block a blood reflux into the inferior vena cava and one to block a blood reflux into the superior vena cava). The manufacturing effort is typically doubled if two different valve entities need to be connected to a stent or any other support structure.

Following the above, in accordance with the invention the gap of the covering can be formed by attaching one or more strip-shaped elements to a support structure in such a way that respective longer edge portions of the strip-shaped elements either overlap or tightly adjoin to one another and/or the gap may be formed by one or more cuts.

Accordingly, in one embodiment, the covering comprises at least one additional longitudinal opening, e.g., an additional cut. Thereby, the at least one additional longitudinal opening extends from a second longitudinal opening starting point to a second longitudinal opening ending point. Like in case of the first longitudinal opening starting point and the first longitudinal opening ending point, the second longitudinal opening starting point and the second longitudinal opening ending point are spaced apart from each other along the longitudinal axis. Furthermore, the first longitudinal opening starting point and the second longitudinal opening starting point are circumferentially spaced apart from each other. Likewise, the first longitudinal opening ending point and the second longitudinal opening ending point are circumferentially spaced apart from each other. This results in at least two longitudinal openings in the covering that are distinct from each other. By applying such longitudinal openings (e.g. two cuts), it is possible to divide the covering into different sections that are movable with respect to each other so that they can provide a gap for a blood flow or close such a gap. Thus, the sections act as valve entities of the artificial cardiac valve.

In accordance with the invention, at least one longitudinal opening (e.g. at least one cut) in the covering is sufficient for basic valve functioning.

However, it turned out that 2 to 10 longitudinal openings, in particular 4 to 6 longitudinal openings, in particular 6 longitudinal openings are particularly appropriate for dividing different sections of the covering from each other. In an embodiment, the covering comprises exactly 2, 4, 6, 8, or 10 longitudinal openings (e.g. cuts).

In an alternative embodiment, 1 to 9 longitudinal openings, in particular 5 to 7 longitudinal openings are appropriate for dividing sections of the covering from each other. In a further alternative embodiment, the covering comprises exactly 1, 3, 5, 7, or 9 longitudinal openings (e.g. cuts).

With the context of the invention, an even number of longitudinal openings (e.g. an even number of cuts) is preferred over an uneven or odd number of longitudinal openings (e.g. an uneven number of cuts).

In an embodiment, the strip-shaped elements and the sections of the covering lying between the strip-shaped elements are symmetrically distributed over the circumference of the support structure. Such an arrangement facilitates implantation of the artificial cardiac valve since no specific orientation of the artificial cardiac valve needs to be observed.

In an embodiment, the strip-shaped elements of the covering and the sections lying between the strip-shaped elements are asymmetrically distributed over the circumference of the support structure. Then, the artificial cardiac valve is, in an embodiment, aligned during implantation such that the desired hemodynamic properties of the artificial cardiac valve result. To give an example, the valve entity of the artificial cardiac valve (formed by two sections of the covering that are movable to each other as disclosed herein) are oriented towards a tricuspid valve or a mitral valve of the patient's heart in order to allow a particularly easy blood flow from the inferior vena cava and/or the superior vena cava or any of the pulmonary veins towards the respective ventricle of the heart.

Even in case of an asymmetric arrangement of the longitudinal openings and the strip-shaped elements of the covering, the same amount of covering material is generally present in each area of the stent. By such an arrangement, the profile of the artificial cardiac valve is not negatively influenced so that an easy implantation is still possible.

In an embodiment, so-called dummy seams (i.e., seams that are only made into the material of the covering without connecting two previously non-connected parts of the covering material together), can be provided to achieve a so-called pseudo-symmetry. Such pseudo-symmetry can counteract any potential difficulties of deployment of the artificial cardiac valve for which a lack of symmetry could be responsible, e.g., asymmetric friction.

Speaking more generally, the covering comprises, in an embodiment, a plurality of additional longitudinal openings. Thereby, each additional longitudinal opening extends from an own longitudinal opening starting point to an own longitudinal opening ending point. Thus, each longitudinal opening starting point is only a starting point for an individual longitudinal opening. Likewise, each longitudinal opening ending point is only an ending point for an individual longitudinal opening. It is possible by such an arrangement that the longitudinal openings extend essentially parallel to each other.

If at least two longitudinal openings are present, a strip-shaped element is formed between these two longitudinal openings. In this scenario then, this strip-shaped element acts as a first section, wherein the sections of the covering on the other sides of the longitudinal openings act as second sections.

In an embodiment, such a strip-shaped element of the covering formed between two adjacent longitudinal openings extends over the full length of the longitudinal openings without interruption. There are in particular no incisions extending in an angled manner with respect to the longitudinal openings that would divide the strip-shaped element into different parts (like a flap). Rather, the strip-shaped element remains a single part of the section of the covering. If more than one strip-shaped element is present due to presence of a plurality of longitudinal openings, all of these strip-shaped elements extend over the full length of the longitudinal openings without interruption, in an embodiment.

In an embodiment, the covering comprises or essentially consists of biological tissue (e.g., pericardium), collagen-based material, cellulose-based material, a polymeric fabric (e.g., a polymeric woven fabric), a polymeric non-woven, a polymeric foil and/or a composite material.

In an embodiment, the biological tissue is prepared biological tissue treated by one or more of the following method steps in any suitable order: decellularization, cross-linking, stabilization, alpha-galactosidase epitope removal.

In an alternative embodiment, it is possible to apply a coating on any of the aforementioned materials to be used for manufacturing the covering in order to enhance the biocompatibility of the covering. Such a coating is in particular provided if the material from which the covering is made is a polymeric material. With this context, in another embodiment a pharmaceutical drug-eluting material may be used for the coating.

In an embodiment, the coating may be applied by any suitable method known to the skilled person in order to increase biocompatibility of the material and/or in order to elute medically advantageous drugs.

In an embodiment, the covering is made in form of a tube. This can be achieved, e.g., by an extrusion technique. Such a manufacturing method can be particularly well applied if the covering is made from a polymeric material. By manufacturing the covering in form of a tube, the overall seam length is reduced so that the manufacturing costs are also reduced.

In an embodiment, the covering is made from a planar material layer that is sewn together with at least one seam to obtain a covering having an overall shape of a substantially cylindric jacket. Such a manufacturing method can also well be applied to coverings made from biologic tissue.

In an alternative embodiment, in case of the use of polymeric materials for the covering, a manufacturing process of the covering starting from a planar material layer can be appropriate.

In an embodiment, the at least one seam extends along the longitudinal axis of the support structure in an area of the covering that does not form a strip-shaped element between two adjacent cuts. Rather, the seam is placed such that it defines an edge of a strip-shaped element or that it extends along a partial section of such an edge of a strip-shaped element. By the avoidance of placing a seam in an area of the covering that forms a strip-shaped element, the formation of an undesired weakness of the strip-shaped element is avoided.

In an embodiment, the seam extends in an area of the covering that is fixed to the support structure so that during intended operation of the artificial cardiac valve, no relative movement of such an area with respect to the support structure occurs. Then, a potential weakness of this area does not play a significant role with respect to the overall stability of the covering or the artificial cardiac valve, respectively.

In an embodiment, the covering comprises tapering end areas. Such tapering end areas facilitate a fixation of the covering to the support structure if the support structure comprises bulges or constrictions. Thus, the tapering end areas enable a specific adjustment of the covering onto the support structure that is to be covered or enwrapped by the covering. The tapering end areas can adapt any desired shape that is particularly well suited for fixing the covering onto the support structure.

In an embodiment, the first longitudinal opening starting point and/or the first longitudinal opening ending point is defined by a recess. This recess can have any desired shape, e.g., a circular shape, an oval shape or an angular shape such as a triangular or a quadrangular shape. Such recess facilitates the fixation of the covering to the support structure and the movability of the covering in case of structural diameter variations of the support structure or temporal diameter changes of the support structure (e.g., during deployment, a support structure such as a stent typically deploys from a transport state in which the stent is long and thin to a deployed state in which the stent is shorter and thicker, i.e., has a bigger diameter). Likewise, the second longitudinal opening starting point and/or the second longitudinal opening ending point as well as any further longitudinal opening starting point and/or any further longitudinal opening ending point can be realized as a recess. Typically, all longitudinal opening starting points and/or all longitudinal opening ending points of a covering have the same shape.

In an embodiment, the longitudinal opening extends straightly along the longitudinal axis. Such a longitudinal opening can be particularly easy to make into the covering. In an embodiment, all longitudinal openings in the covering extend straightly along the longitudinal axis. Thus, they represent a direct, linear connection between their respective longitudinal opening starting point and their respective longitudinal opening ending point.

In an embodiment, the longitudinal opening extends curvedly along the longitudinal axis. By such an arrangement, the relative size of movable sections of the covering (acting like a valve leaflet so that they can also be referred to as valve leaflet) and the relative size of sections fixedly connected to the stent can be modified. Furthermore, a curved longitudinal opening (such as a curved cut) can facilitate, in an embodiment, the abutment of the covering in the area of a varying diameter of the support structure such as a stent, i.e. in a transitional area between a bulged area and a constricted area of the support structure. If more than one longitudinal opening is present in the covering, some or all of these longitudinal opening can curvedly extend along the longitudinal axis. Then, differently shaped movable strip-shaped elements and fixed sections of the covering result.

In an embodiment, the support structure such as a stent comprises a bulge in a first transitional area between the first end area and the intermediate area and/or in a second transition area between the second end area and the intermediate area. The bulge is defined as an area of the stent having a bigger diameter than the surrounding area of the stent. Such a bulge can facilitate an arrangement or a fixation of the artificial cardiac valve in the inferior vena cava or the superior vena cava or one of the pulmonary veins. In particular, if two bulges are present (one in the first transition area and one in the second transition area), an undesired displacement of the artificial cardiac valve at its implantation site is effectively prevented.

In an embodiment, the support structure (e.g. a stent) comprises a plurality of alternating bulges and constrictions. The stent then appears in a longitudinal section as having a wave-shaped outer form. Such a shape enables an increase of the number of valve sections (formed by the first and the second section of the covering) of the artificial cardiac valve and further enables to decrease the individual valve cross sections. This can increase the lifetime of the artificial cardiac valve.

Typically, the support structure has a symmetric outer shape, in particular a shape that is rotationally symmetric with respect to the longitudinal axis of the support structure. Further, different orientations of the support structure during implantation may be possible, provided that a respective orientation does not impart the functionality of the artificial cardiac valve. This is typically the case as long as also the longitudinal openings and/or strip-shaped elements are symmetrically distributed over the circumference of the support structure such as a stent.

With the above context, it shall be noted that a suitable rotational orientation of the implant shall be ensured in any case.

In an embodiment, the stent has an asymmetric outer shape, in particular a rotationally asymmetric outer shape with respect to the longitudinal axis (L) as rotational axis. This enables an adjustment of the stent to the anatomy of the implantation site. In case of such an asymmetric outer shape, marker elements such as radiopaque markers can be present on the support structure and/or on the covering in order to facilitate a correct positioning of the artificial cardiac valve during implantation. Likewise, a radiopaque suture can be used as a marker element.

In an embodiment, the support structure is a self-expanding stent having a grid-like or net-like structure. Such a grid-like or net-like structure can be realized by a plurality of open cells that may be formed by a wire scaffold that optionally includes a braided structure or via suitable laser-cutting techniques (e.g. a laser-cut from an initial tubular structure). Thereby, the individual cells can have different shapes and sizes in order to selectively increase or decrease the stability, flexibility and radial forces of the stent in certain sections. Nitinol or other shape-memory materials are particularly appropriate for manufacturing the stent. For instance, the stent may be laser-cut out of a Nitinol tube.

In an embodiment, the support structure is a mechanically expandable stent such as a balloon-stent.

In an embodiment, the support structure is a stent comprising both a self-expanding and a mechanically expanding part.

In an embodiment, the support structure (e.g. a stent) does not comprise any bifurcations. It rather has an overall elongated outer shape that is yet flexible or bendable.

To achieve a partial or full overlapping of the first section of the covering and the second section of the covering or of a strip-shaped element of the covering with respect to surrounding areas of the covering, and to achieve a particularly simple delivery of the artificial cardiac valve to an implantation site, different possibilities exist:
According to one possibility, the artificial cardiac valve can be present in a compressed delivery state (also referred to as a crimped state). In such a state, the support structure has a relatively high length, but a relatively small diameter. The covering is then arranged around the support structure without significant length excess. The artificial cardiac valve can well be delivered in this delivery state by use of a typical delivery tool such as a delivery catheter, similar to, e.g., a TAVI catheter or a catheter for peripheral stents, and thereby the delivery tool being adapted to the implantation site. Such a delivery catheter typically comprises an inner shaft comprising a prosthesis connector, an outer shaft having a sheath such as a capsule, and optionally further comprising a stabilizer tubing as the outermost sheath structure. In accordance with the invention, the delivery catheter is configured such that it is suitably adapted for the respective implant and the specific site of implantation. Such a delivery catheter can further comprise one or more steering and positioning mechanisms.

It is then released from the delivery tool and transferred from its delivery state to its deployed state (also referred to as expanded state). Upon transition of the artificial cardiac valve from its delivery state to its deployed state, it typically experiences foreshortening, i.e., a decrease of its length connected with an increase of its diameter. The covering is fixed to the stent such that individual sections can rearrange during this transition so that the first section can afterwards overlap the second section of the covering, e.g., a first strip-shaped element of the covering can overlap a second strip-shaped element of the covering being present on the opposite side of the first strip-shaped element and thereby defining a longitudinal opening along the respective longer edges of the first and second strip-shaped elements. Alternatively, a longitudinal opening may be generated by way of a cut in the first and/or second strip-shaped element. In this alternative case, the first and second strip-shaped element not necessarily overlap.

It is also possible to use a covering having an excess length with respect to the length of the support structure. This embodiment is particularly appropriate in case of a stent as support structure having a diameter that does not change over the length of the stent. For bringing an according artificial cardiac valve into its delivery state, the excess length of the covering can be folded onto the stent so as to achieve a profile (overall diameter of the artificial cardiac valve) as small as possible.

In an embodiment, the covering can be fixed with an appropriate fixing means temporarily in this folded position. An appropriate fixing means is, e.g., an easily soluble glue. But generally, such a folding results in an overall bigger diameter than in case of other variants without length excess of the covering.

A higher diameter of the artificial cardiac valve requires also a bigger size of the delivery tool. Generally, a small diameter of the delivery tool is desired since the smaller the diameter of the delivery tool, the better tolerated is an implantation making use of the delivery tool.

In view of all the foregoing disclosure, in further embodiments:
- the longitudinal opening may extend straightly or curvedly along the longitudinal axis,
- the support structure may have an asymmetric outer shape, and/or
- the support structure may not comprise any bifurcations.

In an aspect, the disclosure relates to a medical method for treating tricuspid valve insufficiency or mitral valve insufficiency of a patient in need thereof. This method comprises the steps explained in the following:
a) Advancing an artificial cardiac valve in a delivery state through an appropriate blood vessel into the right atrium or into the left atrium of the patient's heart. Thereby, the artificial cardiac valve comprises a support structure such as a hollow-cylindrical stent having a first end area and a second end area spaced apart from the first end area, the support structure further having an intermediate area extending between the first end area and the second end area along a longitudinal axis (L); and a covering at least partially enwrapping an outer side of the support structure, wherein the covering is connected to the support structure by at least one connection and fluidly separates an interior of the support structure from an outer space of the support structure; wherein the first end area comprises a first opening allowing blood to enter into the interior of the support structure; wherein the second end area comprises a second opening allowing blood to enter into the interior of the support structure; wherein the covering comprises a longitudinal opening; wherein the longitudinal opening extends from a first longitudinal opening starting point to a first longitudinal opening ending point, the first longitudinal opening starting point and the first longitudinal opening ending point being spaced apart from each other along the longitudinal axis (L), wherein the longitudinal opening at least partially extends along the intermediate area of the longitudinal opening and wherein a first section of the covering positioned at a first side of the longitudinal opening and a second section of the covering positioned at a second side of the longitudinal opening are relatively movable to each other and either overlap or tightly adjoin to one another along the longitudinal opening so that the covering can be present:
   i) in a first configuration, in which a gap is present between the first section of the covering and the second section of the covering, thereby the gap allowing an anterograde blood flow from the interior of the support structure towards the outer space of the support structure across the longitudinal opening of the covering, and
   ii) in a second configuration, in which substantially no gap is present between the first section of the covering and the second section of the covering, so that a retrograde blood flow from the outer space of the stent towards the interior of the stent across the longitudinal opening of the covering is prevented by the covering;
b) at least partially deploying the artificial cardiac valve;
c) placing the first end area into a first cardiac blood vessel chosen from the group consisting of the inferior vena cava, the superior vena cava and any of the pulmonary veins;
d) placing the second end area into a second cardiac blood vessel chosen from the group consisting of the inferior vena cava, the superior vena cava and any of the pulmonary veins, wherein the second cardiac blood vessel is different from the first cardiac blood vessel but is located in the same atrium of the patient's heart as the first blood vessel; and
e) fully deploying the artificial cardiac valve if not yet done.

These steps can be carried out in any desired sequence that is suitable for full and functional deployment of the artificial cardiac heart valve at a proper site of implantation.

All embodiments of the artificial cardiac valve can be combined in any desired way and can be transferred in an analogous manner to the described medical method, and vice versa.

Further details of aspects of the present invention will be explained with respect to exemplary embodiments and accompanying Figures. In the Figures:
- Fig. 1A: shows a side view of a first embodiment of an artificial cardiac valve in its delivery state;
- Fig. 1B: shows a cross-sectional view through the artificial cardiac valve of Figure 1A;
- Fig. 1C: shows a side view of the artificial cardiac valve of Figure 1A in its deployed state and open configuration;
- Fig. 1D: shows a cross-sectional view through the artificial cardiac valve of Figure 1C along the line marked with D-D in Figure 1C;
- Fig. 1E: shows a side view of the artificial cardiac valve of Figure 1A in its deployed state and closed configuration;
- Fig. 2A: shows a cut pattern of an embodiment of a covering of an artificial cardiac valve;
- Fig. 2B: shows a side view of a second embodiment of an artificial cardiac valve comprising a covering having a cut pattern as shown in Figure 2A;
- Fig. 2C: shows a cross-sectional view of the artificial cardiac valve of Figure 2B along the dashed line indicated in Figure 2B;
- Fig. 2D: shows a cross-sectional view of the artificial cardiac valve of Figure 2B in its closed configuration at the same site of the artificial cardiac valve;
- Fig. 3A: shows a side view of a third embodiment of an artificial cardiac valve in its delivery state;
- Fig. 3B: shows the artificial cardiac valve of Figure 3A in its deployed state and open configuration;
- Fig. 3C: shows the artificial cardiac valve of Figure 3A in its deployed state and closed configuration;
- Fig. 3D: shows the artificial cardiac valve of Figure 3A in its deployed state and open configuration at an exemplary implantation site;
- Fig. 4A: shows a side view of a fourth embodiment of an artificial cardiac valve;
- Fig. 4B: shows a cut pattern of the covering of the artificial cardiac valve of Figure 4A;
- Fig. 4C: shows a cross-sectional view of the artificial cardiac valve of Figure 4A in its open configuration along the dashed line indicated in Figure 4A;
- Fig. 4D: shows a cross-sectional view of the artificial cardiac valve of Figure 4A in its closed configuration at the same site of the artificial cardiac valve;
- Fig. 5A: shows another embodiment of a cut pattern of a covering of an artificial cardiac valve;
- Fig. 5B: shows a cross-sectional view through an artificial cardiac valve having a covering with a cut pattern as shown in Figure 5A in its open configuration;
- Fig. 5C: shows a cross-sectional view through an artificial cardiac valve having a covering with a cut pattern as shown in Figure 5A in its closed configuration;
- Fig. 6A: shows another embodiment of a cut pattern for a covering of an artificial cardiac valve;
- Fig. 6B: shows another embodiment of a cut pattern of a covering of an artificial cardiac valve;
- Fig. 7A: shows another cut pattern of a covering of an artificial cardiac valve;
- Fig. 7B: shows a cross-sectional view through a covering made from the cut pattern shown in Figure 7A;
- Fig. 8A: shows another embodiment of a cut pattern of the covering of an artificial cardiac valve;
- Fig. 8B: shows a cross-sectional view through a covering made from the cut pattern shown in Figure 8A;
- Fig. 9A: shows another embodiment of a cut pattern of a covering of an artificial cardiac valve;
- Fig. 9B: shows a cross-sectional view through a covering made from the cut pattern shown in Figure 9A;
- Fig. 10A: shows a side view of another embodiment of a stent of an artificial cardiac valve;
- Fig. 10B: shows a side view of another embodiment of a stent of an artificial cardiac valve; and
- Fig. 10C: shows a side view of another embodiment of a stent of an artificial cardiac valve.

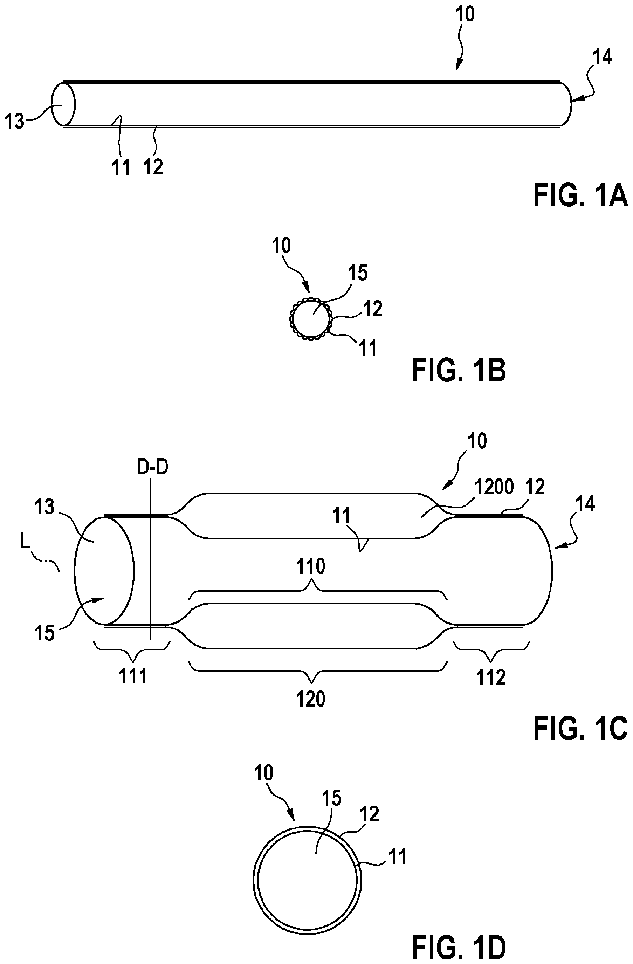

Figure 1A shows a partially cut side view of an artificial cardiac valve 10 comprising a stent 11 as support structure and a covering 12 surrounding said stent 11. Thereby, this artificial cardiac valve 10 is shown in its delivery state in which it has a comparatively high length but a comparatively small diameter.

The artificial cardiac valve 10 has a first opening 13 on its first end and a second opening 14 on its second end. Blood can enter through these openings into an interior of the artificial cardiac valve 10. However, the covering 12 prevents blood from flowing out of the interior of the artificial cardiac valve 10 at other sites than the first opening 13 and the second opening 14. This is only possible through additional openings such as a longitudinal opening in the covering that will be explained in particular with respect to Figure 1C.

Figure 1B shows a cross-sectional view of the artificial cardiac valve 10 of Figure 1A. Here, it can be seen that there is a material excess of the covering 12 surrounding the stent 11 so that the covering 12 is laid around the stent 11 in an unordered manner. Figure 1B also reveals that the stent 11 surrounds an interior 15 of the artificial cardiac valve 10. Figure 1C shows a partially cut side view of the artificial cardiac valve 10 of Figure 1A in its deployed state. Thereby, the same numeral references will be used here and in all following Figures for the same elements of the same embodiments.

The artificial cardiac valve 10 can be present in its deployed state in two configurations. In the first configuration, the so-called open configuration, a first section 120 of the covering 12 is lifted away from the stent 11 as support structure and from another section of the covering 12 that is not visible in the depiction of Figure 1C. The relative movement of the first section 120 of the covering 12 with respect to the other section of the covering 12 as well with respect to the stent 11 is due to a longitudinal opening in the covering 12 (e.g. a cut) that extends along a longitudinal axis L of the artificial cardiac valve 10. Thereby, this longitudinal opening extends in an intermediate area 110 of the stent 11 that is located between a first end area 111, in which the first opening 13 is positioned, and a second end area 112 in which the second opening 14 is positioned.

When the first section 120 of the covering 12 is lifted away from the stent 11, a gap 1200 is formed between the first section 120 of the covering 12 and the stent 11 as well as between other sections of the covering 12. Thereby, it is possible for blood that has entered the interior 15 of the artificial cardiac valve 10 through the first opening 13 or through the second opening 14 to exit this interior 15 through the gap 1200.

Figure 1D shows a cross-sectional view through the artificial cardiac valve 10 of Figure 1C in its deployed state along the line D-D located in the first end area 111 of the stent 11 as support structure. Due to stent deployment, there is no material excess of the covering 12 with respect to the stent 11, so that the covering 12 surrounds or enwraps the stent 11 in a single layer. A material excess is still present in a central area of the artificial cardiac valve due to a decreased diameter of the stent 11 and a sufficiently big dimension of the first section 120 of the covering 12.

Figure 1D shows another partially cut side view of the artificial cardiac valve in its deployed state. Thereby, the second configuration of the artificial cardiac valve 10, i.e., the closed configuration, is shown. In this closed configuration, the full covering 12, i.e., also the first section 120 of the covering 12 abuts against the stent 11 so that no gap is present any longer. This configuration is typically taken by the covering 12 when an external pressure acts upon the covering 12 from outside of the artificial cardiac valve 10. In such a case, substantially no fluid flow, in particular substantially no blood flow, is possible through the covering 12. Thus, the artificial cardiac valve 10 acts like a check valve and enables a blood flow from its interior 15 through the gap 1200 (cf. Figure 1C) to an outside of the artificial cardiac valve 10 (antegrade blood flow), but prevents blood flow in the opposite direction (retrograde blood flow). The blood pressure relationships within the heart determine whether the artificial cardiac valve is open or closed.

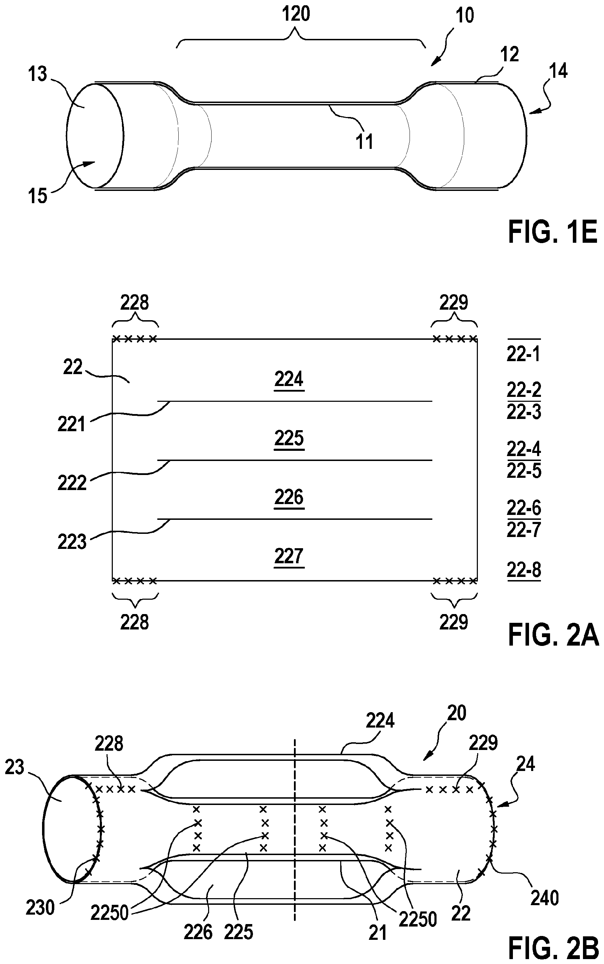

Figure 2A shows a cut pattern of the covering 22 comprising a first cut 221, a second cut 222 and a third cut 223 as an exemplary longitudinal opening of the invention. Due to these cuts 221, 222, 223, the covering 22 is divided into a first section 224, a second section 225, a third section 226 and a fourth section 227. Each of these sections 224, 225, 226, 227 can also be referred to as strip-shaped elements and have a first edge and a second edge, wherein always a second edge of one section lies directly opposite a first edge of an adjacent section with respect to the cut extending between those sections. Therewith, the covering 22 comprises eight section edges in total, namely the first section edge 22-1, the second section edge 22-2, the third section edge 22-3, the fourth section edge 22-4, the fifth section edge 22-5, the sixth section edge 22-6, the seventh section edge 22-7, and the eighth section edge 22-8.

The covering 22 further comprises a first connection section 228 and a second connection section 229. In both connection sections 228, 229, a seam is to be provided (indicated by "xxxx") so that the covering 22 can be rolled like a cylinder jacket and sewn together with two seams, one in the first connection section 228 and one of the second connection section 229. The section between the first connection section 228 and the second connection section 229 will not be sewn. Rather, this section of the covering 22 will be left open and constitutes a cut-like element, even though it is not necessary to provide a cut at this site. Thus, the second section 227 is then directly adjacent to the first section 224, but still be movable with respect to the first section 224. Such an arrangement minimizes the sewing effort, reduces the manufacturing costs and the possibilities of manufacturing errors. This leads to an increase in product safety.

Figure 2B shows an artificial cardiac valve 20 which comprises a stent 21 as a support structure and a covering 22 that is prepared according to the cut pattern of Figure 2A. Thereby, the first connection section 228 and the second connection section 229 are sewn together with one seam in each case. Furthermore, the covering 22 is sewn to the stent 21 in a circumference of a first opening 23 by a first seam 230 acting as first connection and in the circumference of a second opening 24 by a second seam 240 acting as second connection. The first section 224 and the third section 226 of the covering 22 are not sewn to the stent 21. Rather, they can act as valve leaflets and can move with respect to the stent 21 and with respect to the second section 225 of the covering 22 and the fourth section 227 of the covering 22. A plurality of seams 2250 is present to secure the second section 225 of the covering 22 tightly to the stent 21. Likewise, a plurality of seams is present to secure the fourth section 227 to the covering 21 (not visible in the depiction of Figure 2B). If required, the covering may be affixed to the stent at other suitable points of attachment, optionally being loosely and not tightly affixed thereto.

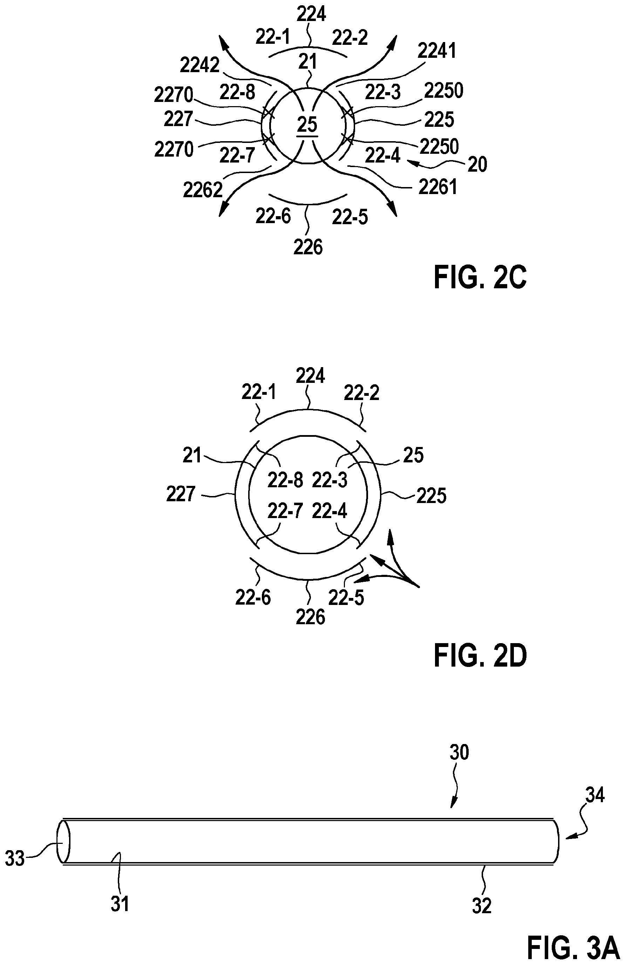

Figure 2C is a cross-sectional view through the artificial cardiac valve 20 of Figure 2B along the dashed line in Figure 2B in the open configuration of the artificial cardiac valve 20. Here, exemplarily, it can well be seen that the first section 224 of a strip-shaped element of the covering 22 partially overlaps with the second section 225 of a strip-shaped element of the covering 22 and the fourth section 227. Likewise, and exemplarily, the third section 226 of a strip-shaped element of the covering 22 partially overlaps with the second section 225 of a strip-shaped element of the covering 22 and the fourth section 227 of a strip-shaped element of the covering 22. Between the first section 224 and the second section 225, a first gap 2241 is formed through which blood can flow from an interior 25 of the artificial cardiac valve 20 to an outside thereof. Likewise, a second gap 2242 is formed between the first section 224 and the fourth section 227. Furthermore, a third gap 2261 is formed between the third section 226 and the second section 225. Finally, a fourth gap 2262 is formed between the third section 226 and the fourth section 227. Blood can flow from the interior 25 of the artificial cardiac valve 20 through all of these gaps 2241, 2242, 2261, 2262 towards an exterior of the artificial cardiac valve 20 (antegrade blood flow). This is indicated by corresponding arrows.

To better visualize a concordance between the cross-sectional view of Figure 2C and the cut pattern depicted in Figure 2A, the individual section edges of the first section 224, the second section 225, the third section 226 and the fourth section 227 of the covering 22 are provided with the respective numeral reference 22-1 to 22-8.

Figure 2D shows another cross-sectional view through the artificial cardiac valve 20 of Figure 2B along the dashed line of Figure 2B. Thereby, the artificial cardiac valve is shown in its second configuration, i.e., in its closed configuration. As can be seen from Figure 2D, there are substantially no gaps present between the first section 224 and either of the second section 225 and the fourth section 227. Furthermore, substantially no gap is present any longer between the third section 226 and either of the second section 225 and the fourth section 227. Therefore, substantially no blood can enter the interior 25 of the artificial cardiac valve 20 from an outside (substantially no retrograde blood flow). This is also exemplarily visualized by according arrows. Once again, the section edges are marked with the respective numeral reference 22-1 to 22-8 to better illustrate the structure of the artificial cardiac valve 20.

Figure 3A shows a partially cut side view of an artificial cardiac valve 30 in its delivery state. It comprises a stent 31 as support structure and a covering 32 surrounding an outside of the stent 31. Furthermore, a first opening 33 and a second opening 34 allow for blood inflow into an interior 35 of the artificial cardiac valve 30. Thereby, the structure of this artificial cardiac valve 30 resembles the structure of the artificial cardiac valve 10 depicted in Figure 1A.

If the artificial cardiac valve 30 of Figure 3A is deployed, foreshortening of the stent occurs, i.e., the length of the stent 31 reduces, whereas the diameter of the stent 31 increases. Furthermore, it is only apparent in the deployed state of the artificial cardiac valve 30 that the stent 31 has a structure differing from the structure of the stent 11 of the artificial cardiac valve 10 of the embodiment shown in Figure 1A. To be more precise, the stent 31 of the artificial cardiac valve 30 comprises a first bulge 311 and a second bulge 312. The first bulge 311 is located in a transition zone between a first end area 313 and an intermediate area 314 of the stent 31. Likewise, the second bulge 312 is located between a second end area 315 and the intermediate area 314 of the stent 31.

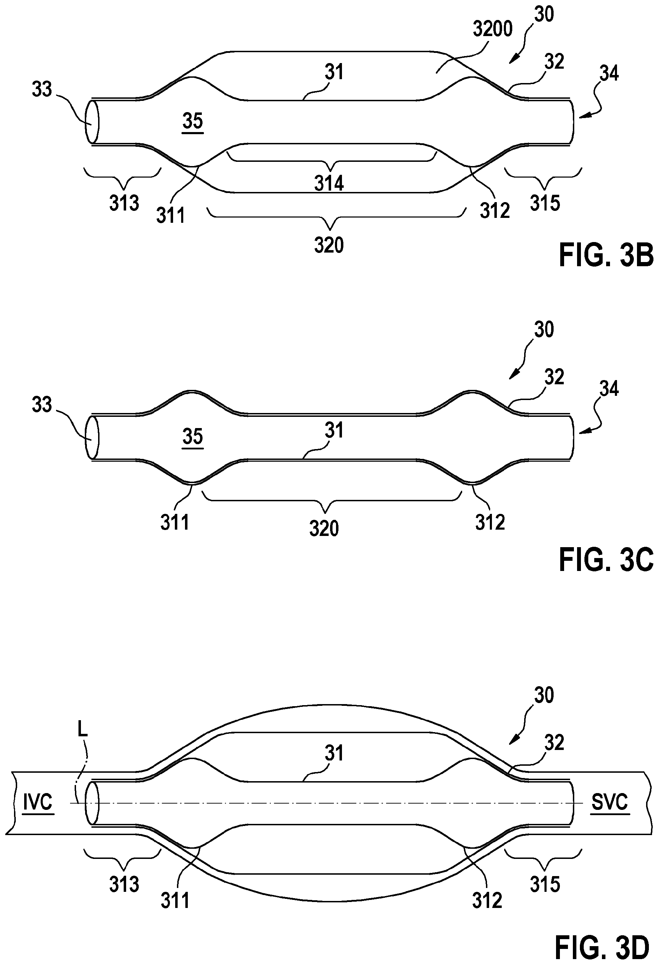

The artificial cardiac valve 30 is depicted in Figure 3B in its open configuration (first configuration), in which an intermediate section 320 of the covering 32 is lifted from the stent 31 so that a gap 3200 is formed between the first section 320 of the covering 32 and the stent 31.

Blood can flow through this gap 3200 from an interior 35 of the artificial cardiac valve 30 to an outside thereof.

Figure 3C shows a partially cut side view of the artificial cardiac valve 30 in its closed configuration (second configuration). As already explained with respect to the embodiment depicted in Figure 1E, the covering 32 - also with its first section 320 - abuts against the stent 31 so that substantially no gap is any longer present. Therefore, substantially no blood can cross the covering 32 from the interior 35 of the artificial cardiac valve 30 to an outside thereof.

Figure 3D shows a schematic sketch of an implantation situation of the artificial cardiac valve 30 shown in Figures 3A to 3C. Thereby, the first end area 313 is inserted into the inferior vena cava (IVC) of a patient. Likewise, the second end area 315 of the artificial cardiac valve 30 is inserted into the superior vena cava (SVC) of the patient. Thereby, the first bulge 311 and the second bulge 312 serve for a good positioning of the artificial cardiac valve 30 between the inferior vena cava (IVC) and the superior vena cava (SVC) and prevent an undesired dislocation of the artificial cardiac valve. At the implantation site between the inferior vena cava (IVC) and the superior vena cava (SVC) of the patient, the artificial cardiac valve 30 extends essentially along its longitudinal axis L.

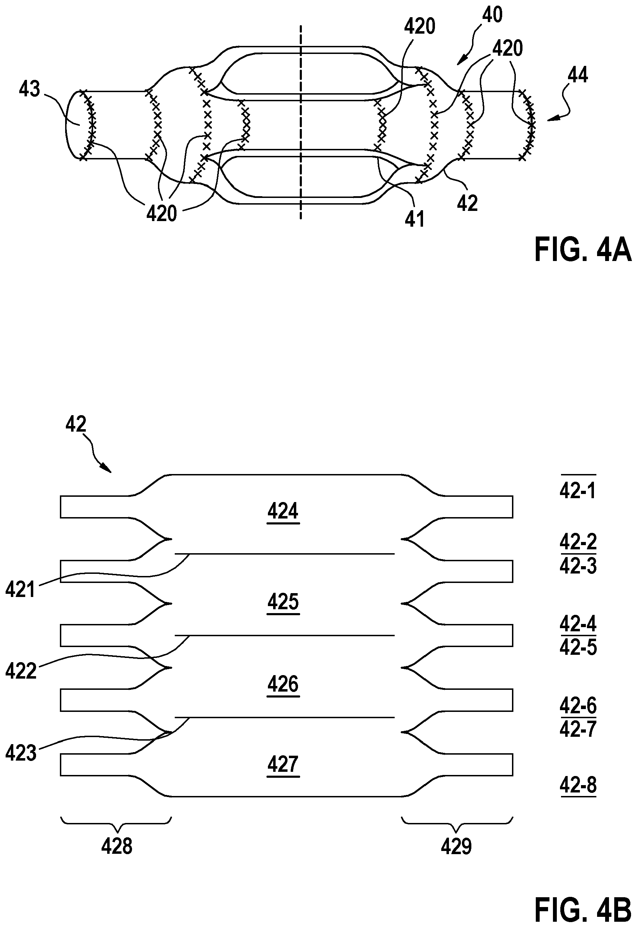

Figure 4A shows a side view of another embodiment of an artificial cardiac valve 40 in accordance with the present invention. Whereas the general functioning of this artificial cardiac valve 40 is similar to the function of the previously described embodiments of artificial cardiac valves, it has a more complex stent structure that allows an even easier fixation of the artificial cardiac valve at an implantation site, in particular in an inferior vena cava and a superior vena cava of a patient.

A covering 42 is connected to a stent 41 as support structure by a plurality of seams 420 in different areas of the stent 41 or the covering 42, respectively. The seams should ensure an optimal attachment of the covering to the stent. Further or additionally, seams are also possible to add at certain points, with which the covering is slidably attached to the stent, so that the covering can occupy an energetically favorable position with little tension in the material when crimping and expanding during delivery. Thus, there are not only seams in the area of a first opening 43 and a second opening 44, but also in intermediate areas. This increases, however, the efforts to be made for sewing the covering 42 to the stent 41.

The cut pattern of the covering 42 is also more complex in this embodiment. This cut pattern is depicted in Figure 4B. The covering 42 comprises a first cut 421, a second cut 422, and a third cut 423 in order to generate a longitudinal opening. These cuts divide the covering 42 into a first section 424, a second section 425, a third section 426 and a fourth section 427 or respective strip-shaped elements.

In an embodiment of the invention, the covering comprises a three-dimensional outer shape. In an embodiment of the invention, the covering comprises a three-dimensional outer shape that protrudes from the surface of the coverage towards the surrounding anatomical site.

In this exemplary embodiment, each of these sections 424, 425, 426, 427 (strip-shaped elements) comprises a first tapering end area and a second tapering end area. The individual first tapering end areas are collectively marked with the numeral reference 428, whereas the individual second tapering end areas are collectively marked with the numeral reference 429.

Each of the sections 424, 425, 426, 427 (strip-shaped elements) comprises a first edge and a second edge. Thus, the covering 42 has eight section edges in total, namely a first section edge 42-1, a second section edge 42-2, a third section edge 42-3, a fourth section edge 42-4, a fifth section edge 42-5, a sixth section edge 42-6, a seventh section edge 42-7, and an eighth section edge 42-8. Thereby, always a second edge of one section lies directly opposite a first edge of an adjacent section across the respective cut being present between the sections. It should be noted that an end area of the fourth section 427 will be sewn to an end area of the first section 424 in order to give the covering 42 the shape of a cylinder jacket. The central area between the fourth section 427 and the first section 424 will not be sewn together so that a cut remains between these two sections. Thus, the first section 424 is movable with respect to the fourth section 427. Thereby, it is not necessary to make a distinct cut between these sections, because it will be automatically present if the sections are not sewn together over the entire width of the covering 42.

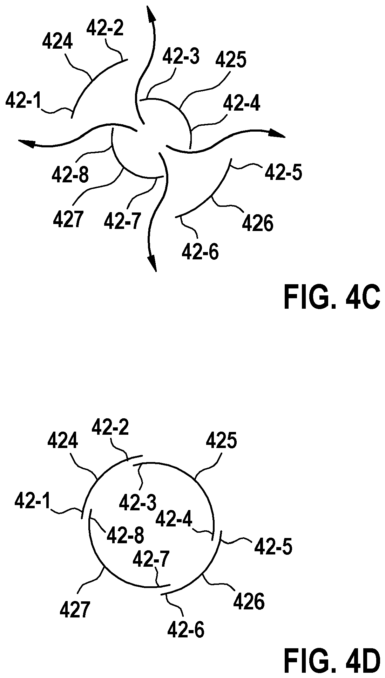

Figure 4C shows a cross-sectional view through the artificial cardiac valve 40 of Figure 4A along the dashed line of Figure 4A. Thereby, the general functioning of the covering 42 and its individual sections 424, 425, 426, 427 is very similar to the overall functioning of the covering 22 that was explained with respect to Figures 2C and 2D. Therefore, reference is made to the explanations of those Figures.

Figure 4D shows a cross-sectional view through the artificial cardiac valve 40 in its closed configuration (second configuration). Once again, reference is made to the explanations given above with respect to Figure 2D that can also be applied in an analogous manner to Figure 4D.

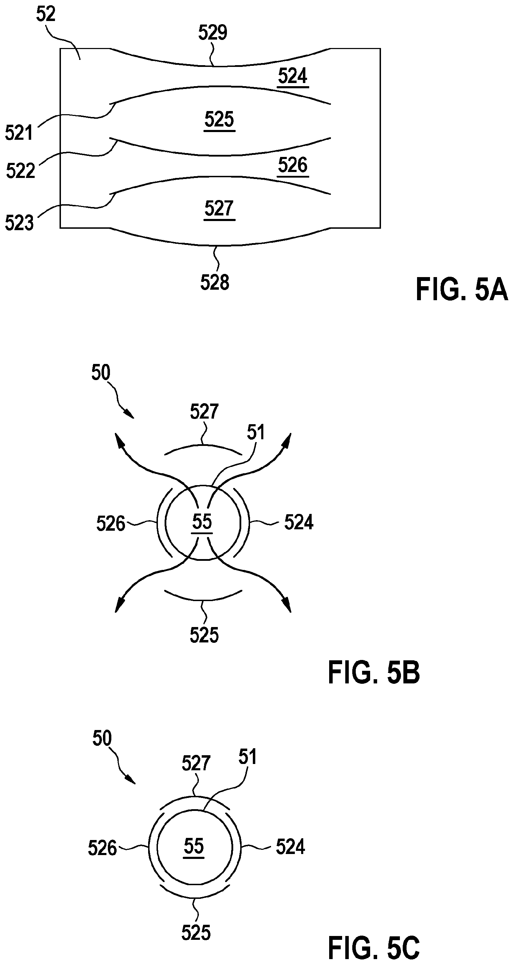

Figure 5A shows a covering 52 for an artificial cardiac valve that does not comprise straight cuts in order to generate a longitudinal opening, but rather three curved cuts, namely a first cut 521, a second cut 522, and a third cut 523. Due to these cuts, a first section 524, a second section 525, a third section 526, and a fourth section 527 are formed or respective strip-shaped elements. These sections 524, 525, 526, 527 have different sizes. Therefore, it is possible to adjust the relative size of movable parts of the covering 52 and of immovable parts thereof to allow for more flexibility when manufacturing an artificial cardiac valve. This configuration also allows for greater longitudinal openings for the respective blood flow therethrough. Furthermore, variations in diameter of the support structure that is to be covered by the covering 52 can be better covered by the covering 52 due to the curved cuts. The covering 52 is intended to be sewn together in order to get the shape of a cylinder jacket. Therefore, also a lower edge 528 and an upper edge 529 of the covering 52 have a curved shape so that all cuts (including a non-sewn area between the lower edge 528 and the upper edge 529 of the covering 52 that has the function of a cut) have the same appearance.

Figure 5B shows a cross-sectional view through an artificial cardiac valve 50 in its open configuration (first configuration). It comprises a stent 51 as support structure and a covering 52 according to the cut pattern shown in Figure 5A. The smaller sections of the covering 52, i.e., the first section 524 and the third section 526, are sewn to the stent 51. The larger sections, i.e., the second section 525 and the fourth section 527 of the covering 52, can lift and act as valve leaflets. Then, bigger gaps are formed between the movable sections 525, 527 and the fixed sections 524, 526 allowing a higher blood flow from an interior 55 of the artificial cardiac valve 50 to an outside thereof (antegrade blood flow).

Figure 5C shows a cross-sectional view through the artificial cardiac valve 50 in its closed configuration (second configuration). In this configuration, substantially no gap is present between the individual sections 524 to 527 of the covering 52, so that substantially no blood flow is possible between an outside of the artificial cardiac valve 50 and its interior 55. Reference is made also to the explanations given with respect to Figure 2D and 4D that are also applicable for the embodiment shown in Figure 5C.

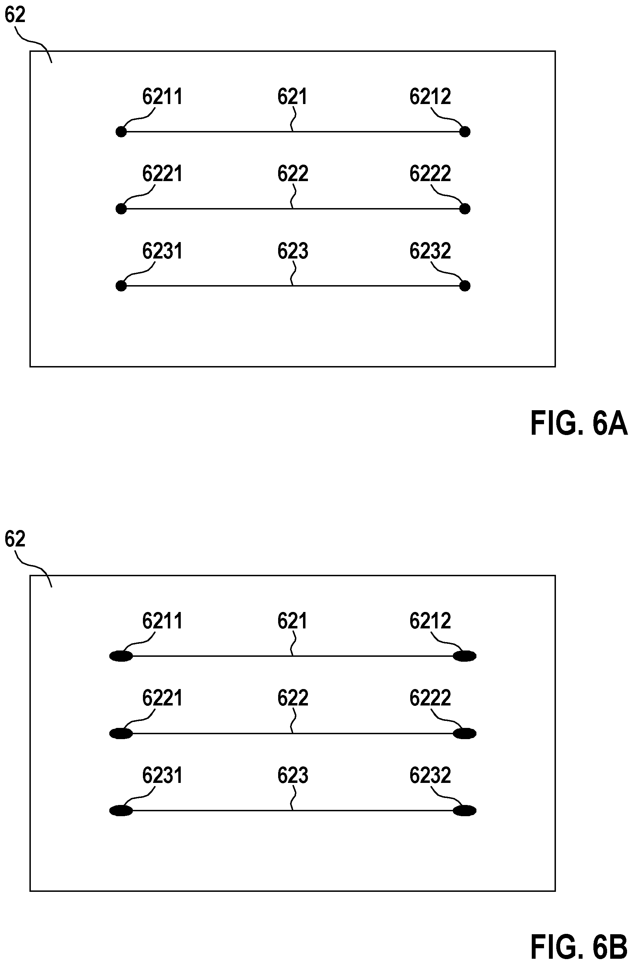

Figure 6A shows an exemplary embodiment of a specific arrangement of longitudinal openings of a covering 62 (e.g. a respective cut pattern). Thereby, the covering 62 comprises three longitudinal openings such as three respective cuts, namely a first cut 621, a second cut 622, and a third cut 623. The first cut 621 extends from a first cut starting point 6211 to a first cut ending point 6212. Likewise, the second cut 622 extends from a second cut starting point 6221 to second cut ending point 6222. Finally, the third cut 623 extends from a third cut starting point 6231 to a third cut ending point 6232. Thereby, the cut starting points 6211, 6221, 6231 and the cut ending points 6212, 6222, 6232 have the form of a circular recess. Such a form increases the mobility of the covering and facilitates the connection of the covering to a stent. Furthermore or additionally, such a form reduces the risk of any rupture to the cuts.

Figure 6B shows another embodiment of the covering 62 that is essentially identical with the embodiment shown in Figure 6A. The only difference is that the cut starting points 6211, 6221, 6231 and the cut ending points 6212, 6222, 6232 are realized in the form of an elliptical recess. It is also possible to combine cut starting points and/or cut ending points of different shapes with each other. Thus, it is possible that some cut starting points and/or some cut ending points have a circular shape, whereas other cut starting points and/or other cut ending points have an elliptic shape. Furthermore, other shapes are also possible.

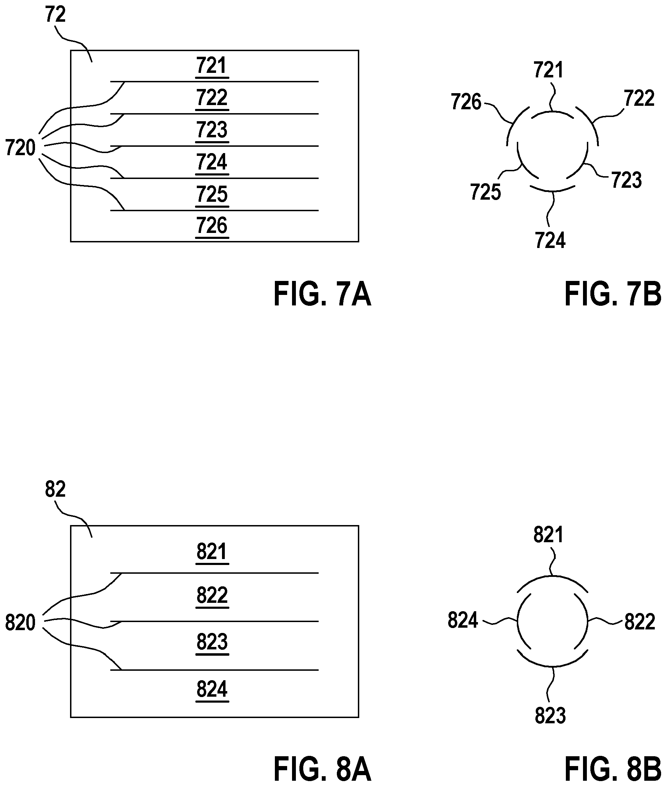

Figure 7A exemplarily shows another embodiment of a specific arrangement of longitudinal openings of a covering 72 such as a respective cut pattern having five cuts 720 and thus comprising six sections or strip-shaped elements in total, namely a first section 721, a second section 722, a third section 723, a fourth section 724, a fifth section 725 and a sixth section 726 or respective strip-shaped elements. Three of these sections can be sewn to the stent, whereas the remaining three sections can act as valve leaflets. A schematic cross-sectional view through an according arrangement is shown in Figure 7B, wherein only the open configuration of an according artificial cardiac valve is depicted.

Figure 8A shows another embodiment of a specific arrangement of longitudinal openings of a covering 82 such as a respective cut pattern comprising three cuts 820 and thus having four sections or strip-shaped elements in total, namely a first section 821, a second section 822, a third section 823, and a fourth section 824 or respective strip-shaped elements. In such a configuration, it is appropriate to sew two of these sections tightly to the stent of an artificial cardiac valve, whereas the remaining two sections can be left movable with respect to the sewn sections so that they can act as valve leaflets.

A schematic cross-sectional view through an according configuration of a covering suited to be used for an artificial cardiac valve is shown in Figure 8B. Thereby, only the open configuration of an according artificial cardiac valve is illustrated (first configuration).

The individual sections or strip-shaped elements of coverings 72 and 82 of Figures 7A to 8 B are typically equally distributed over the circumference of a support structure such as a stent of an according cardiac valve. This results in a symmetric appearance of the respective cardiac valve. However, also an asymmetric distribution of the individual sections or strip-shaped elements is generally possible.

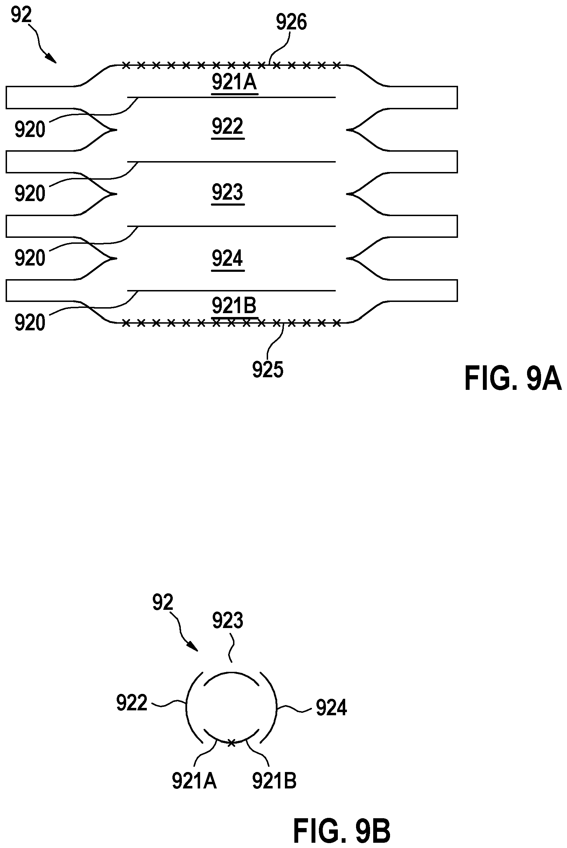

Figure 9A shows another embodiment of a specific arrangement of longitudinal openings of a covering 92 such as a respective cut pattern intended to be used for an artificial cardiac valve. Thereby, the overall shape of this cut pattern resembles the shape of the cut pattern illustrated in Figure 4B. Therefore, reference is made to the explanations given with respect to Figure 4B.

A difference between the cut pattern of the covering 92 of Figure 9A and the cut pattern of the covering 42 of Figure 4B is that the individual cuts 920 of the covering 92 are located in a different area of the covering 92 than the first cut 421, the second cut 422 and the third cut 423 of the covering 42 depicted in Figure 4B are.

Due to the four individual cuts 920, five sections or respective strip-shaped elements, namely a first section 921A, a second section 922, a third section 923, a fourth section 924 and a fifth section 921B are formed. When the planar layer of the covering 92 is rolled to form a cylinder jacket and sewn along its lower edge 925 and its upper edge 926, the first section 921A and the fifth section 921B are combined to form a common section. Thus, all in all four sections result also in this embodiment.

One section, namely the common section of first section 921A and the fifth section 921B comprises a seam along its longitudinal axis. Since such a seam represents a structural weakness of the respective section, it is particularly appropriate if the common section of the first section 921A and the fifth section 921B is not a movable section acting as valve leaflet, but is rather a section that is sewn to a stent of the respective cardiac valve. An according schematic cross-sectional view through a valve configuration making use of the covering 92 is depicted in Figure 9B. Thereby, the covering 92 is shown in its open configuration (first configuration).

It should be noted that the embodiment depicted in Figures 9A and 9B requires an additional cut and a longer seam than other embodiments. It results in a potential structural weakness due to the seam (marked with "xxxxx") between the first section 921A and the fifth section 921B.

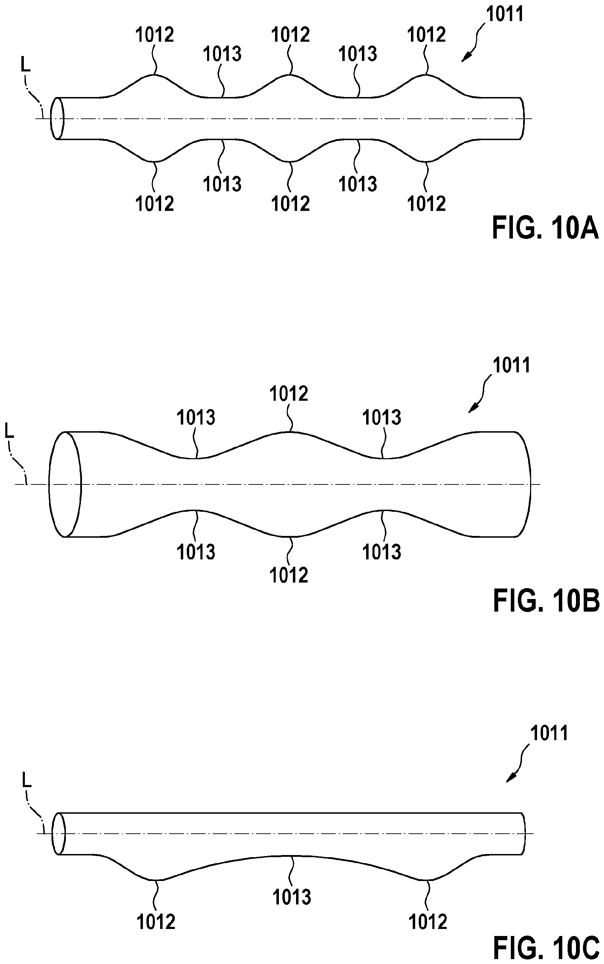

Figures 10A to 10C show different alternative shapes of a stent 1011 to be used as a support structure for an artificial cardiac valve of the invention. Thereby, the embodiment shown in Figure 10A is a stent 1011 comprising a plurality of bulges 1012 and constrictions 1013. This stent 1011 has an overall rotationally symmetric shape with respect to its longitudinal axis L serving as rotational axis. It has a wave-like shape in a longitudinal section. However, in accordance with the present invention, also other numbers of bulges and constrictions are possible. The stent 1011 shown in Figure 10B has only one central bulge 1012 with two adjoining constrictions 1013. It also has an overall rotationally symmetric shape with respect to its longitudinal axis L. However, in accordance with the present invention, also other numbers of bulges and constrictions are possible.

Figure 10C shows an embodiment of the stent 1011 that has an asymmetric outer shape, i.e., that is not rotationally symmetric with respect to its longitudinal axis L. Such a shape can be used to allow an adaptation of an artificial cardiac valve making use of the stent 1011 suitable to specific anatomic requirements. If the stent 1011 has such an asymmetric appearance, it can be helpful if the stent 1011 or an insertion catheter used for delivering the stent to the implantation site comprises one or more marker elements in order to identify and/or correct the orientation of an artificial cardiac valve comprising the stent 1011.

## Claims

1. Artificial cardiac valve, comprising:
a support structure (11) having a first end area (111) and a second end area (112) spaced apart from the first end area (111), the support structure (11) further having an intermediate area (110) extending between the first end area (111) and the second end area (112) along a longitudinal axis (L); and
a covering (12) at least partially enwrapping an outer side of the support structure (11), wherein the covering (12) is connected to the support structure (11) by at least one connection (2250) and fluidly separates an interior (15) of the support structure (11) from an outer space of the support structure (11);
wherein the first end area (111) comprises a first opening (13) allowing blood to enter into the interior of the support structure (11); and
wherein the second end area (112) comprises a second opening (14) allowing blood to enter into the interior of the support structure (11); and
wherein the covering (12) comprises a longitudinal opening (221);
wherein the one longitudinal opening (221) extends from a first longitudinal opening starting point (6211) to a first longitudinal opening ending point (6212), the first longitudinal opening starting point (6211) and the first longitudinal opening ending point (6212) being spaced apart from each other along the longitudinal axis (L),
wherein the longitudinal opening (221) at least partially extends along the intermediate area (110) of the support structure (11), and
wherein a first section (120) of the covering (12) positioned at a first side of the longitudinal opening (221) and a second section (225) of the covering (12) positioned at a second side of the longitudinal opening (221) are relatively movable to each other and either overlap or tightly adjoin to one another along the longitudinal opening (221), so that the covering (12) is configured to be present in two different configurations:
i) in a first configuration, in which a gap (1200) is present between the first section (120) of the covering (12) and the second section (225) of the covering (12), thereby the gap (1200) allowing an anterograde blood flow from the interior (15) of the support structure (11) towards the outer space of the support structure (11) across the longitudinal opening of the covering (12), and
ii) in a second configuration, in which substantially no gap is present between the first section (120) of the covering (12) and the second section (225) of the covering (12), so that a retrograde blood flow from the outer space of the support structure (11) towards the interior (15) of the support structure (11) across the longitudinal opening of the covering (12) is prevented by the covering (12),
**characterized in that**
the covering (12) is formed by at least two strip-shaped elements, wherein the at least two strip-shaped elements are arranged on the support structure such that they allow for the formation of the longitudinal opening (221) between the at least two strip-shaped elements, and wherein the at least two strip-shaped elements are further arranged such that the longitudinal opening extends at least over a partial length of the strip-shaped elements (221) without interruption, preferably within the intermediate area (110).

2. Artificial cardiac valve according to claim 1, **characterized in that** the support structure (11) is a stent.

3. Artificial cardiac valve according to claim 2, **characterized in that** the stent is a braided wire stent or a wire frame stent, or a combination thereof, or a laser-cut stent.

4. Artificial cardiac valve according to any one of the preceding claims, **characterized in that** the covering (12) comprises at least one additional longitudinal opening (221), wherein the at least one additional longitudinal opening (221) extends from a second longitudinal opening starting point (6221) to a second longitudinal opening ending point (6222), the second longitudinal opening starting point (6221) and the second longitudinal opening ending point (6222) being spaced apart from each other along the longitudinal axis (L), wherein the first longitudinal opening starting point (6211) and the second longitudinal opening starting point (6221) are circumferentially spaced apart from each other, and wherein the first longitudinal opening ending point (6212) and the second longitudinal opening ending point (6222) are circumferentially spaced apart from each other.

5. Artificial cardiac valve according to any one of the preceding claims, **characterized in that** the covering comprises a plurality of additional longitudinal openings (221), wherein each additional longitudinal opening (221) extends from an own longitudinal opening starting point (6211) to an own longitudinal opening ending point (6212).

6. Artificial cardiac valve according to any one of the preceding claims, **characterized in that** the support structure (31, 1011) comprises a bulge (311, 1012) in a first transition area between the first end area (313) and the intermediate area (314) and/or in a second transition area between the second end area (315) and the intermediate area (314) or the support structure (31, 1011) comprises a plurality of alternating bulges (311, 1012) and constrictions (1013).

7. Artificial cardiac valve according to any one of the claims 1 to 5, **characterized in that** the covering (12) is formed by at least one strip-shaped element (120), wherein a cut in the strip-shaped element forms the longitudinal opening (221), and wherein the cut is arranged in the covering such that the longitudinal opening extends at least over a partial length of the strip-shaped element (221) without interruption, preferably within the intermediate area (110).

8. Artificial cardiac valve according to any one of the preceding claims, **characterized in that** the covering (12) comprises or essentially consists of a biological tissue, such as pericardium, cellulose, collagen, a polymeric fabric, a polymeric non-woven, a polymeric foil, and/or a composite material.

9. Artificial cardiac valve according to any one of the preceding claims, **characterized in that** the covering (12) is made from a planar material layer that is sewn together with at least one seam to obtain an overall shape of a cylinder jacket.

10. Artificial cardiac valve according to any one of the claims 1 to 8, **characterized in that** the covering (12) is made from a tubular material.

11. Artificial cardiac valve according to any one of the claims 1 to 8, **characterized in that** the covering (12) is made from a three-dimensionally-shaped material, the three-dimensionally-shaped material being optionally sewn together with at least one seam to obtain an overall shape of a cylinder jacket provided that the three-dimensionally-shaped material itself is not a tubular material.

12. Artificial cardiac valve according to any one of the claims 9 to 11, **characterized in that** the at least one seam extends along the longitudinal axis (L) in an area of the covering (12) that does not form a strip-shaped element (120) between two adjacent longitudinal openings (221).

13. Artificial cardiac valve according to any one of the preceding claims, **characterized in that** the covering (12) comprises tapering end areas (428).

14. Artificial cardiac valve according to any one of the preceding claims, **characterized in that** the first longitudinal opening starting point (6211) and/or the first longitudinal opening ending point (6212) is defined by a recess.

15. Artificial cardiac valve according to any one of the preceding claims for use in assisting or replacing tricuspid valve functionality or mitral valve functionality.

## Patentansprüche

1. Künstliche Herzklappe, enthaltend:
eine Stützstruktur (11) mit einem ersten Endbereich (111) und einem zweiten Endbereich (112), der von dem ersten Endbereich (111) beabstandet ist, wobei die Stützstruktur (11) ferner einen Zwischenbereich (110) aufweist, der sich zwischen dem ersten Endbereich (111) und dem zweiten Endbereich (112) entlang einer Längsachse (L) erstreckt; und
eine Abdeckung (12), die zumindest teilweise eine Außenseite der Stützstruktur (11) umhüllt, wobei die Abdeckung (12) mit der Stützstruktur (11) durch mindestens eine Verbindung (2250) verbunden ist und einen Innenraum (15) der Stützstruktur (11) von einem Außenraum der Stützstruktur (11) fluidisch trennt;
wobei der erste Endbereich (111) eine erste Öffnung (13) aufweist, die den Eintritt von Blut in das Innere der Stützstruktur (11) ermöglicht; und
wobei der zweite Endbereich (112) eine zweite Öffnung (14) aufweist, die den Eintritt von Blut in das Innere der Stützstruktur (11) ermöglicht; und
wobei die Abdeckung (12) eine Längsöffnung (221) aufweist;
wobei sich die eine Längsöffnung (221) von einem ersten Längsöffnungsstartpunkt (6211) zu einem ersten Längsöffnungsendpunkt (6212) erstreckt, wobei der erste Längsöffnungsstartpunkt (6211) und der erste Längsöffnungsendpunkt (6212) entlang der Längsachse (L) voneinander beabstandet sind,
wobei sich die Längsöffnung (221) zumindest teilweise entlang des Zwischenbereichs (110) der Stützstruktur (11) erstreckt, und
wobei ein erster Abschnitt (120) der Abdeckung (12), der an einer ersten Seite der Längsöffnung (221) positioniert ist, und ein zweiter Abschnitt (225) der Abdeckung (12), der an einer zweiten Seite der Längsöffnung (221) positioniert ist, relativ zueinander beweglich sind und sich entweder überlappen oder eng aneinander entlang der Längsöffnung (221) angrenzen, so dass die Abdeckung (12) so konfiguriert ist, dass sie in zwei verschiedenen Konfigurationen vorliegt:
i) in einer ersten Konfiguration, in der ein Spalt (1200) zwischen dem ersten Abschnitt (120) der Abdeckung (12) und dem zweiten Abschnitt (225) der Abdeckung (12) vorhanden ist, wodurch der Spalt (1200) einen anterograden Blutfluss vom Inneren (15) der Stützstruktur (11) zum Außenraum der Stützstruktur (11) über die Längsöffnung der Abdeckung (12) ermöglicht, und
ii) in einer zweiten Konfiguration, in der im Wesentlichen kein Spalt zwischen dem ersten Abschnitt (120) der Abdeckung (12) und dem zweiten Abschnitt (225) der Abdeckung (12) vorhanden ist, so dass ein retrograder Blutfluss vom Außenraum der Stützstruktur (11) zum Inneren (15) der Stützstruktur (11) über die Längsöffnung der Abdeckung (12) durch die Abdeckung (12) verhindert wird,
**dadurch gekennzeichnet, dass**
die Abdeckung (12) durch mindestens zwei streifenförmige Elemente gebildet wird, wobei die mindestens zwei streifenförmigen Elemente so an der Stützstruktur angeordnet sind, dass sie die Ausbildung der Längsöffnung (221) zwischen den mindestens zwei streifenförmigen Elementen ermöglichen, und wobei die mindestens zwei streifenförmigen Elemente ferner so angeordnet sind, dass sich die Längsöffnung zumindest über eine Teillänge der streifenförmigen Elemente (221) ohne Unterbrechung, vorzugsweise innerhalb des Zwischenbereichs (110), erstreckt.

2. Künstliche Herzklappe nach Anspruch 1, **dadurch gekennzeichnet, dass** die Stützstruktur (11) ein Stent ist.

3. Künstliche Herzklappe nach Anspruch 2, **dadurch gekennzeichnet, dass** der Stent ein geflochtener Drahtstent oder ein Drahtrahmenstent oder eine Kombination davon oder ein lasergeschnittener Stent ist.

4. Künstliche Herzklappe nach einem der vorhergehenden Ansprüche, **dadurch gekennzeichnet, dass** die Abdeckung (12) mindestens eine zusätzliche Längsöffnung (221) aufweist, wobei sich die mindestens eine zusätzliche Längsöffnung (221) von einem zweiten Längsöffnungsstartpunkt (6221) zu einem zweiten Längsöffnungsendpunkt (6222) erstreckt, der zweite Längsöffnungs-Startpunkt (6221) und der zweite Längsöffnungs-Endpunkt (6222) entlang der Längsachse (L) voneinander beabstandet sind, wobei der erste Längsöffnungs-Startpunkt (6211) und der zweite Längsöffnungs-Startpunkt (6221) in Umfangsrichtung voneinander beabstandet sind, und wobei der erste Längsöffnungs-Endpunkt (6212) und der zweite Längsöffnungs-Endpunkt (6222) in Umfangsrichtung voneinander beabstandet sind.

5. Künstliche Herzklappe nach einem der vorhergehenden Ansprüche, **dadurch gekennzeichnet, dass** die Abdeckung eine Vielzahl von zusätzlichen Längsöffnungen (221) aufweist, wobei sich jede zusätzliche Längsöffnung (221) von einem eigenen Längsöffnungs-Anfangspunkt (6211) zu einem eigenen Längsöffnungs-Endpunkt (6212) erstreckt.

6. Künstliche Herzklappe nach einem der vorhergehenden Ansprüche, **dadurch gekennzeichnet, dass** die Stützstruktur (31, 1011) in einem ersten Übergangsbereich zwischen dem ersten Endbereich (313) und dem Zwischenbereich (314) und/oder in einem zweiten Übergangsbereich zwischen dem zweiten Endbereich (315) und dem Zwischenbereich (314) eine Ausbuchtung (311, 1012) aufweist oder die Stützstruktur (31, 1011) eine Vielzahl von abwechselnden Ausbuchtungen (311, 1012) und Engstellen (1013) aufweist.

7. Künstliche Herzklappe nach einem der Ansprüche 1 bis 5, **dadurch gekennzeichnet, dass** die Abdeckung (12) durch mindestens ein streifenförmiges Element (120) gebildet ist, wobei ein Einschnitt in dem streifenförmigen Element die Längsöffnung (221) bildet, und wobei der Einschnitt so in der Abdeckung angeordnet ist, dass sich die Längsöffnung zumindest über eine Teillänge des streifenförmigen Elements (221) ohne Unterbrechung, vorzugsweise innerhalb des Zwischenbereichs (110), erstreckt.

8. Künstliche Herzklappe nach einem der vorhergehenden Ansprüche, **dadurch gekennzeichnet, dass** die Abdeckung (12) ein biologisches Gewebe, wie Perikard, Zellulose, Kollagen, ein polymeres Gewebe, ein polymeres Vlies, eine polymere Folie und/oder ein Verbundmaterial umfasst oder im Wesentlichen daraus besteht.

9. Künstliche Herzklappe nach einem der vorhergehenden Ansprüche, **dadurch gekennzeichnet, dass** die Abdeckung (12) aus einer flächigen Materialschicht besteht, die mit mindestens einer Naht zusammengenäht ist, um eine Gesamtform eines Zylindermantels zu erhalten.

10. Künstliche Herzklappe nach einem der Ansprüche 1 bis 8, **dadurch gekennzeichnet, dass** die Abdeckung (12) aus einem rohrförmigen Material besteht.

11. Künstliche Herzklappe nach einem der Ansprüche 1 bis 8, **dadurch gekennzeichnet, dass** die Abdeckung (12) aus einem dreidimensional geformten Material hergestellt ist, wobei das dreidimensional geformte Material gegebenenfalls mit mindestens einer Naht zusammengenäht ist, um eine Gesamtform eines Zylindermantels zu erhalten, vorausgesetzt, dass das dreidimensional geformte Material selbst kein schlauchförmiges Material ist.

12. Künstliche Herzklappe nach einem der Ansprüche 9 bis 11, **dadurch gekennzeichnet, dass** die mindestens eine Naht entlang der Längsachse (L) in einem Bereich der Abdeckung (12) verläuft, der kein streifenförmiges Element (120) zwischen zwei benachbarten Längsöffnungen (221) bildet.

13. Künstliche Herzklappe nach einem der vorhergehenden Ansprüche, **dadurch gekennzeichnet, dass** die Abdeckung (12) sich verjüngende Endbereiche (428) aufweist.

14. Künstliche Herzklappe nach einem der vorhergehenden Ansprüche, **dadurch gekennzeichnet, dass** der erste Längsöffnungs-Anfangspunkt (6211) und/oder der erste Längsöffnungs-Endpunkt (6212) durch eine Ausnehmung definiert ist.

15. Künstliche Herzklappe nach einem der vorhergehenden Ansprüche zur Verwendung als Unterstützung oder zum Ersatz der Trikuspidalklappenfunktion oder der Mitralklappenfunktion.

## Revendications

1. Valve cardiaque artificielle, comprenant :
une structure de support (11) ayant une première zone d'extrémité (111) et une deuxième zone d'extrémité (112) espacée de la première zone d'extrémité (111), la structure de support (11) ayant en outre une zone intermédiaire (110) s'étendant entre la première zone d'extrémité (111) et la deuxième zone d'extrémité (112) le long d'un axe longitudinal (L) ; et
un revêtement (12) enveloppant au moins partiellement un côté extérieur de la structure de support (11), dans lequel le revêtement (12) est relié à la structure de support (11) par au moins une connexion (2250) et sépare de manière fluide un intérieur (15) de la structure de support (11) d'un espace extérieur de la structure de support (11) ;
dans laquelle la première zone d'extrémité (111) comprend une première ouverture (13) permettant au sang de pénétrer à l'intérieur de la structure de support (11) ; et
dans laquelle la deuxième zone d'extrémité (112) comprend une deuxième ouverture (14) permettant au sang de pénétrer à l'intérieur de la structure de support (11) ; et
dans lequel le revêtement (12) comprend une ouverture longitudinale (221) ;
dans laquelle l'ouverture longitudinale (221) s'étend d'un premier point de départ d'ouverture longitudinale (6211) à un premier point d'arrivée d'ouverture longitudinale (6212), le premier point de départ d'ouverture longitudinale (6211) et le premier point d'arrivée d'ouverture longitudinale (6212) étant espacés l'un de l'autre le long de l'axe longitudinal (L),
dans lequel l'ouverture longitudinale (221) s'étend au moins partiellement le long de la zone intermédiaire (110) de la structure de support (11), et
dans lequel une première section (120) de la revêtement (12) positionnée sur un premier côté de l'ouverture longitudinale (221) et une seconde section (225) de la revêtement (12) positionnée sur un second côté de l'ouverture longitudinale (221) sont relativement mobiles l'une par rapport à l'autre et se chevauchent ou se joignent étroitement l'une à l'autre le long de l'ouverture longitudinale (221), de sorte que la revêtement (12) est configurée pour être présente dans deux configurations différentes :
i) dans une première configuration, dans laquelle un espace (1200) est présent entre la première section (120) de la revêtement (12) et la deuxième section (225) de la revêtement (12), l'espace (1200) permettant ainsi un flux sanguin antérograde de l'intérieur (15) de la structure de support (11) vers l'espace extérieur de la structure de support (11) à travers l'ouverture longitudinale de la revêtement (12), et
ii) dans une deuxième configuration, dans laquelle il n'y a pratiquement pas d'espace entre la première section (120) de la revêtement (12) et la deuxième section (225) de la revêtement (12), de sorte que la revêtement (12) empêche un flux sanguin rétrograde de l'espace extérieur de la structure de support (11) vers l'intérieur (15) de la structure de support (11) à travers l'ouverture longitudinale de la revêtement (12),
**caractérisé par le fait que**
le revêtement (12) est formé par au moins deux éléments en forme de bande, dans lequel les au moins deux éléments en forme de bande sont disposés sur la structure de support de manière à permettre la formation de l'ouverture longitudinale (221) entre les au moins deux éléments en forme de bande, et dans lequel les au moins deux éléments en forme de bande sont en outre disposés de manière à ce que l'ouverture longitudinale s'étende au moins sur une longueur partielle des éléments en forme de bande (221) sans interruption, de préférence à l'intérieur de la zone intermédiaire (110).

2. Valve cardiaque artificielle selon la revendication 1, **caractérisée en ce que** la structure de support (11) est un stent.

3. Valve cardiaque artificielle selon la revendication 2, **caractérisée en ce que** le stent est un stent en fil tressé ou un stent en cadre de fil, ou une combinaison de celles-ci, ou un stent découpée au laser.

4. Valve cardiaque artificielle selon l'une quelconque des revendications précédentes, **caractérisée en ce que** le revêtement (12) comprend au moins une ouverture longitudinale supplémentaire (221), dans laquelle l'au moins une ouverture longitudinale supplémentaire (221) s'étend d'un deuxième point de départ d'ouverture longitudinale (6221) à un deuxième point d'arrivée d'ouverture longitudinale (6222), le deuxième point de départ de l'ouverture longitudinale (6221) et le deuxième point d'arrivée de l'ouverture longitudinale (6222) sont espacés l'un de l'autre le long de l'axe longitudinal (L), le premier point de départ de l'ouverture longitudinale (6211) et le deuxième point de départ de l'ouverture longitudinale (6221) étant espacés l'un de l'autre de manière circonférentielle, et le premier point d'arrivée de l'ouverture longitudinale (6212) et le deuxième point d'arrivée de l'ouverture longitudinale (6222) étant espacés l'un de l'autre de manière circonférentielle.

5. Valve cardiaque artificielle selon l'une quelconque des revendications précédentes, **caractérisée en ce que** le revêtement comprend une pluralité d'ouvertures longitudinales supplémentaires (221), où chaque ouverture longitudinale supplémentaire (221) s'étend d'un point de départ d'ouverture longitudinale propre (6211) à un point d'arrivée d'ouverture longitudinale propre (6212).

6. Valve cardiaque artificielle selon l'une quelconque des revendications précédentes, **caractérisée en ce que** la structure de support (31, 1011) comprend un renflement (311, 1012) dans une première zone de transition entre la première zone d'extrémité (313) et la zone intermédiaire (314) et/ou dans une deuxième zone de transition entre la deuxième zone d'extrémité (315) et la zone intermédiaire (314) ou la structure de support (31, 1011) comprend une pluralité de renflements (311, 1012) et de rétrécissements (1013) alternés.

7. Valve cardiaque artificielle selon l'une quelconque des revendications 1 à 5, **caractérisée en ce que** le revêtement (12) est formé par au moins un élément en forme de bande (120), dans lequel une découpe de l'élément en forme de bande forme l'ouverture longitudinale (221), et dans lequel la découpe est disposée dans le revêtement de telle sorte que l'ouverture longitudinale s'étend au moins sur une longueur partielle de l'élément en forme de bande (221) sans interruption, de préférence à l'intérieur de la zone intermédiaire (110).

8. Valve cardiaque artificielle selon l'une quelconque des revendications précédentes, **caractérisée en ce que** le revêtement (12) comprend ou est essentiellement constitué d'un tissu biologique, tel que le péricarde, la cellulose, le collagène, un tissu polymère, un non-tissé polymère, une feuille polymère, et/ou un matériau composite.

9. Valve cardiaque artificielle selon l'une quelconque des revendications précédentes, **caractérisée en ce que** le revêtement (12) est constitué d'une couche de matériau plane qui est cousue avec au moins une couture pour obtenir une forme globale de chemise cylindrique.

10. Valve cardiaque artificielle selon l'une quelconque des revendications 1 à 8, **caractérisée en ce que** le revêtement (12) est constitué d'un matériau tubulaire.

11. Valve cardiaque artificielle selon l'une quelconque des revendications 1 à 8, **caractérisée en ce que** le revêtement (12) est réalisé dans un matériau de forme tridimensionnelle, le matériau de forme tridimensionnelle étant éventuellement assemblé par au moins une couture pour obtenir une forme globale de chemise cylindrique, à condition que le matériau de forme tridimensionnelle ne soit pas lui-même un matériau tubulaire.

12. Valve cardiaque artificielle selon l'une quelconque des revendications 9 à 11, **caractérisée en ce que** la au moins une couture s'étend le long de l'axe longitudinal (L) dans une zone du revêtement (12) qui ne forme pas un élément en forme de bande (120) entre deux ouvertures longitudinales adjacentes (221).

13. Valve cardiaque artificielle selon l'une quelconque des revendications précédentes, **caractérisée en ce que** le revêtement (12) comprend des zones d'extrémité effilées (428).

14. Valve cardiaque artificielle selon l'une quelconque des revendications précédentes, **caractérisée en ce que** le point de départ de la première ouverture longitudinale (6211) et/ou le point d'arrivée de la première ouverture longitudinale (6212) est défini par un renfoncement.

15. Valve cardiaque artificielle selon l'une des revendications précédentes pour l'utilisation dans l'assistance ou le remplacement de la fonctionnalité de la valve tricuspide ou de la valve mitrale.
